# EUROPEAN PATENT APPLICATION

(11) **EP 3 964 511 A1**
(43) Date of publication of application: **09.03.2022**
(21) Application number: 20382788.6
(22) Date of filing: 04.09.2020
(51) Int. Cl.: C07D 487/04, A61B 5/00, A61K 49/00, A61K 31/4192

(54) **BIOORTHOGONAL CHOLESTEROL PROBE AND USES THEREOF**

(71) Applicant: Universidad del País Vasco/Euskal Herriko Unibertsitatea, 48940 Leoia, Bikaia (ES)
(72) Inventor: CONTRERAS GÓMEZ, Xabier, 48940 Leioa - Vizcaya (ES); LORIZATE NOGALES, Maier, 48940 Leioa - Vizcaya (ES)
(74) Representative: ABG Intellectual Property Law, S.L.

(57) **Abstract**

The invention relates to the use of a compound of formula (I), in combination with a cycloalkyne compound labeled with a detectable agent, for detecting cholesterol and/or monitoring cholesterol dynamics in cell membranes

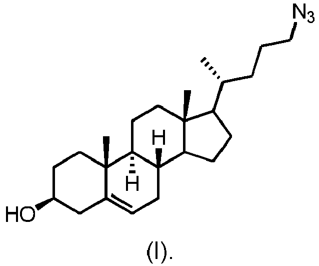

## Description

### FIELD OF THE INVENTION

The invention is comprised within the field of biotechnology and biomedicine. It specifically relates to the use of a bioorthogonal smart probe for detecting cholesterol in cellular membranes.

### BACKGROUND OF THE INVENTION

Cholesterol (chol) regulates many membrane-associated processes, including membrane organization, endocytosis, and transmembrane signaling, through direct interaction with different cellular components. Although several indirect experiments point to the existence of specific chol-dependent lipid nanodomains (often referred to as *lipid rafts*), such nanostructures are still controversial owing, in part, to the difficulty to probe membrane nanoscale heterogeneity by high-resolution nanoscopy in living cells. The discrepancies arise mainly from the presumed size of such structures (5-200 nm), which is below the diffraction limit used by conventional detection methods (>250 nm). Current super-resolution fluorescence microscopy techniques have, by far, overcome this diffraction limit and allow for direct visualization of cellular processes at the nanoscale in living cells. However, direct visualization of lipid rafts in unperturbed cell membranes has not been achieved yet. Unlike protein clusters, which have been trapped and visualized, direct observation of lipid nanodomains is hampered by the rapid diffusion of lipids within cell membranes. Moreover, the imaging of chol at higher levels of organization (i.e., tissues and organs) has been elusive. In the brain, for instance, although the chol distribution has substantial effects, current methods fail to offer a detailed picture showing this cause-effect relationship.

Defects in chol metabolism, levels, and function have been related to a wide range of neurodegenerative diseases (i.e., Alzheimer disease (AD), Parkinson) and mental disorders such as depression. Measuring chol dynamics and distribution in cells and organs at higher resolution is a daunting task that requires the development of novel labeling strategies to generate functional lipid derivatives apt to super-resolution nanoscopy (SRN) and in-depth tissue or in living cells studies.

While there has been significant progress in the study of chol dynamics and trafficking in cultured cells using chol analogs covalently linked to extrinsic fluorophores, the bulky adducts present in these cholesterol derivatives have an enormous impact on its chemical structure and often results in biased membrane interactions. Filipin, a membrane-permeable and intrinsically fluorescent antibiotic, has been used to bind free chol in membranes and monitor chol dynamics in cells. This method, however, presents some disadvantages since (1) the labeling has to be performed in fixed cells because it perturbs cellular membranes and (2) is rapidly photobleached.

Recently, chol analogs based on copper-catalyzed alkyne-azide cycloaddition (CuAAC) and containing a terminal alkyne have been synthesized and used for chol imaging. For example, Hofmann et al. (Journal of Lipid Research 2014, 55, 583), Gaebler et al. (Journal of Lipid Research 2016, 57, 1934) and Jao et al. (Chembiochem. 2015, 16, 611) disclose methods for imaging cholesterol through the use of a copper-catalyzed click chemistry reaction between an alkyne cholesterol derivative and a reporter azide. Nonetheless, copper toxicity and the requirement of a fixation step for intracellular molecular labeling (due to the presence of endogenous copper-binding proteins that block the CuAAC reaction) render them useless for tracking biomolecules in complex living cells or systems *in vivo.*

There is still a need for chol probes able to preserve the physicochemical properties of the natural molecule and suitable for *in vivo* and *in situ* detection.

### SUMMARY OF THE INVENTION

The inventors have found that cholesterol analogue (I) (also denoted herein as Chol-N₃ or compound of formula (I)) completely mimics the biophysical, biochemical and metabolical properties of chol and follows the same intracellular uptake pathway. This compound is therefore suitable for detecting cholesterol and for monitoring cholesterol dynamics.

Cholesterol analogue (I) reacts with labeled cycloalkynes under Cu-free click-chemistry reaction conditions to give rise to an adduct suitable for in situ visualization. Therefore, the combination of cholesterol analogue (I) with a labeled cycloalkyne provides a new probe for in situ detection of chol.

The inventors have confirmed that neither compound of formula (I) nor treatment with the labeled cycloalkyne exerts any toxic effect, which confirms that the invention is suitable for application in living cells and in *in vivo* systems.

The invention provides a very versatile method for detection of chol; different visualization techniques can be used, depending on the desired purpose just by changing the label attached to the cycloalkyne.

Additionally, the chol probe of the invention allows the use of super-resolution nanoscopy for detecting or visualizing chol nanodomains with a very high-resolution.

Furthermore, the inventors have found that the probe of the invention can be also used above the cellular level, for imaging tissues, complete organs or even organisms.

Consequently, cholesterol analogue (I) allows for highly efficient, specific, reliable and easy cholesterol detection, since it completely mimics endogenous cholesterol and can be detected even in very low amounts.

Therefore, in a first aspect the invention is directed to a method for detecting cholesterol in cell membranes comprising:
i) administering to a cell population or to an organism a compound of formula (I) 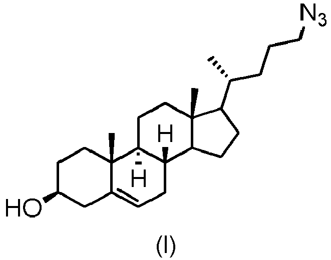 under conditions adequate for the inclusion of said compound in the cell membranes of the cell population or in the cell membranes of the cells of the organism;
ii) contacting the cell population or the cells of the organism of step i) with a cycloalkyne compound labeled with a detectable agent; and
iii) detecting the presence of the detectable agent in the cell membranes.

In a second aspect, the invention relates to a method for monitoring cholesterol dynamics within the cell membranes of a cell population or within the cell membranes of the cells of an organism, which comprises detecting cholesterol according to a method as defined in the first aspect.

In a third aspect, the invention relates to the use of a compound of formula (I) as defined herein, for detecting cholesterol or monitoring cholesterol in cell membranes *in vitro*, *ex vivo or in vivo*, wherein the compound of formula (I) is used in combination with a cycloalkyne compound labeled with a detectable agent.

In a fourth aspect, the invention relates to a compound of formula (I), for use in a method of diagnosis *in vivo* of a cell membrane related disease in a subject in need thereof, wherein the compound of formula (I) is used in combination with a cycloalkyne compound labeled with a detectable agent.

In a fifth aspect, the invention is directed to an *in vitro* or ex *vivo* method of diagnosis of a cell membrane related disease, in a subject in need thereof, wherein the method comprises the use of a compound of formula (I) as defined herein in combination with a cycloalkyne compound labeled with a detectable agent.

In a sixth aspect, the invention is directed to an adduct obtained by reacting a compound of formula (I) and a cycloalkyne compound labeled with a detectable agent.

A seventh aspect of the invention is directed to a kit comprising a compound of formula (I) and a cycloalkyne compound labeled with a detectable agent.

Further aspects of the invention refer to the use of the adduct or the kit of the invention for detecting cholesterol or for monitoring cholesterol within cell membranes.

### DESCRIPTION OF THE FIGURES

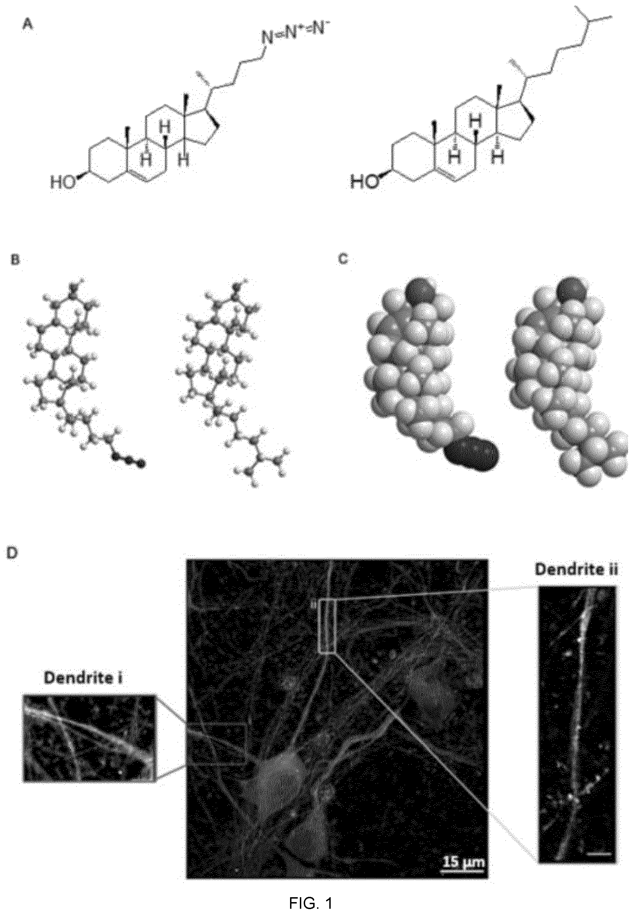

**Figure 1****.** *Structure and characterization of chol-N₃.* (A) Schematic representation of the cholesterol smart probe (chol-N₃) and chol. (B, C) Molecular modeling structures of chol-N₃ and chol: (B) Ball&Stick structure and (C) space filling structure of chol-N₃ vs. chol. Lowest energy structures in vacuum were computed using CS Chem3D Ultra software. (D) Chol-N₃ organization in living cortical neurons. Neurons were grown in the presence of chol-N₃, subjected to Cu-free cycloaddition reaction with AF 546-DBCO, and visualized by confocal microscopy. Representative sections of chol-N₃ distribution in neural dendrites are presented at higher magnification in image boxes (i, ii). Image representative of n=4 independent experiments.

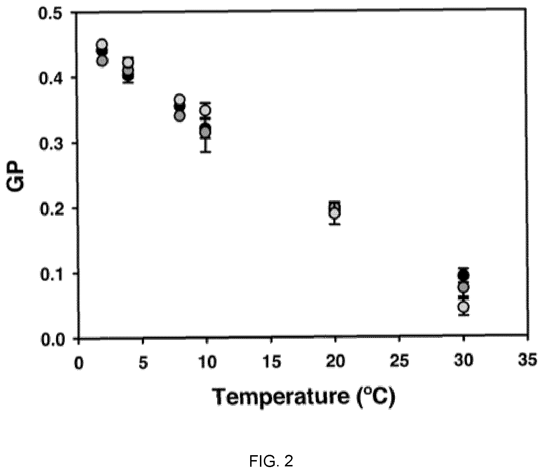

**Figure 2****.** Effect of chol-N₃ and chol-AF 647-DBCO on LUV membrane order. Comparison of temperature-dependent GP profiles of chol (black), chol-N₃ (dark grey) and labeled AF 647-DBCO-chol (light grey). LUVs composed of POPC:chol (90%:10%), POPC: chol-N₃ (90%:10%) and POPC: chol-N₃: chol-N₃-AF 647-DBCO (90%:8%:2%) were stained with 5 µM laurdan and analysed as described in experimental procedures. GP values were calculated from emission spectra for each temperature. Data represent the mean ± SD of n=3 replicates.

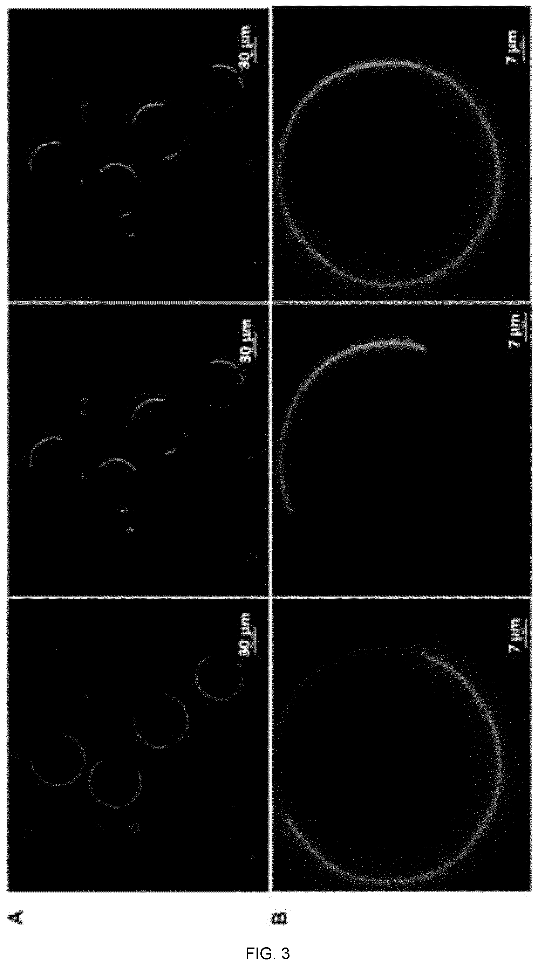

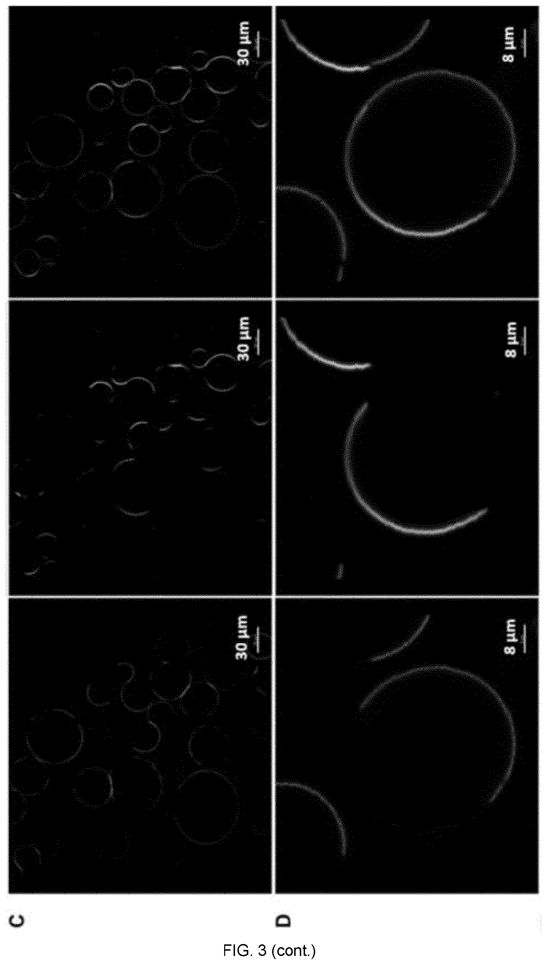

**Figure 3****.** *Partitioning characteristics of the chol-N₃ analog compared to chol in phase-separated GUVs.* (A) overall confocal images of the equatorial plane of GUVs composed of DOPC/SM/chol (3:3:1, mol/mol) and co-labeled with 0.2% Rho-DHPE (left panel) and 0.5% of NAP (middle panel) probes that label the Ld phase and the Lo phase, respectively. Overlay images of two different channels are shown in the right panel. (B): Representative confocal zoom images of the equatorial plane of GUVs composed as described in A, Ld-marker Rho-DHPE (left panel), Lo-marker NAP (middle panel) and overlay images (right panel). (C) overall confocal images of the equatorial plane of GUVs composed of DOPC/SM/chol-N₃ (3:3:1, mol/mol) and co-labeled with 0.2% Rho-DHPE (left panel), and 0.5% of NAP (middle panel) and merge channels (right panel). (D) representative confocal zoom images of the equatorial plane of GUVs composed as described in C, Ld-marker Rho-DHPE (left panel), Lo-marker NAP (middle panel) and overlay channels (right panel). Representatives images of n=3 independent experiments.

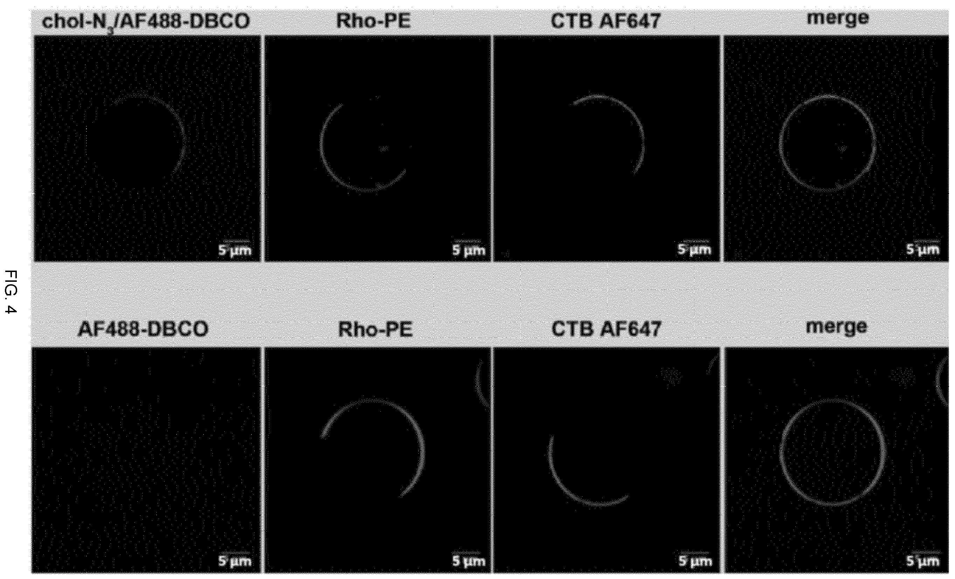

**Figure 4****.** *Partitioning characteristics of in situ labeled chol-N₃ analog in phase-separated GUVs.* Confocal images of the equatorial plane of GUVs composed of DOPC/SM/chol-N₃ (3:3:1, mol/mol) (top panel) and DOPC/SM/chol (3/3/1) (low panel) containing 0.2% Rho-DHPE (Ld marker) and 1% of ganglioside (GM1). Cholera Toxin B AF 647 (CTB AF 647) was added externally binding to GM1 and labeling the Lo-phase. AF 488-DBCO was added externally to labeled 1% of chol-N₃ present in the membrane. Representatives images of n=3 independent experiments.

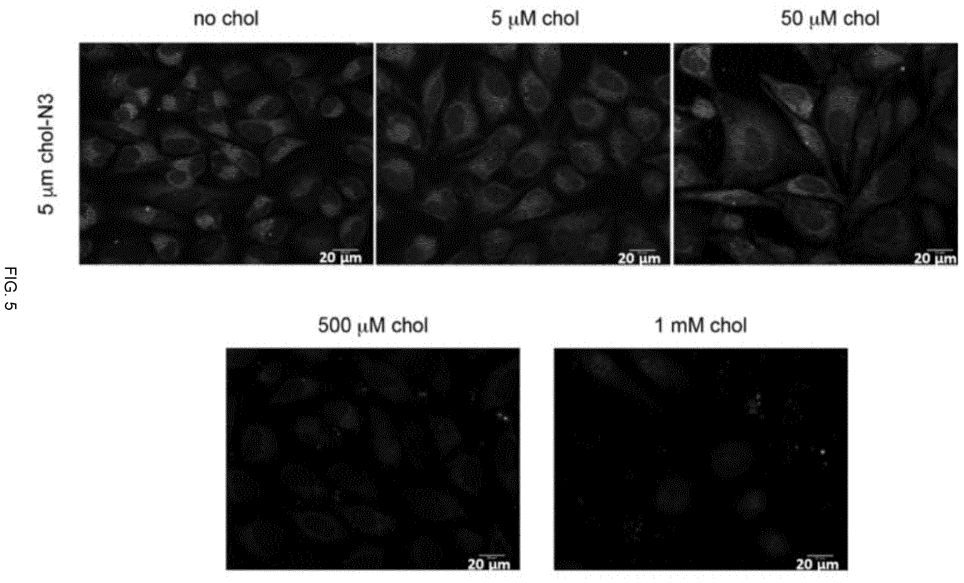

**Figure 5****.** *Competition* assay *to characterize chol-N₃ uptake in cells.* HeLa cells were co-incubated with a fixed concentration (5 µM) of chol-N₃ and increasing concentration of chol (0-1 mM) in cell culture media. After incubation, the cells were fixed and stained with AF 488-DBCO and counterstained with DAPI. Representative images of n=4 independent experiments.

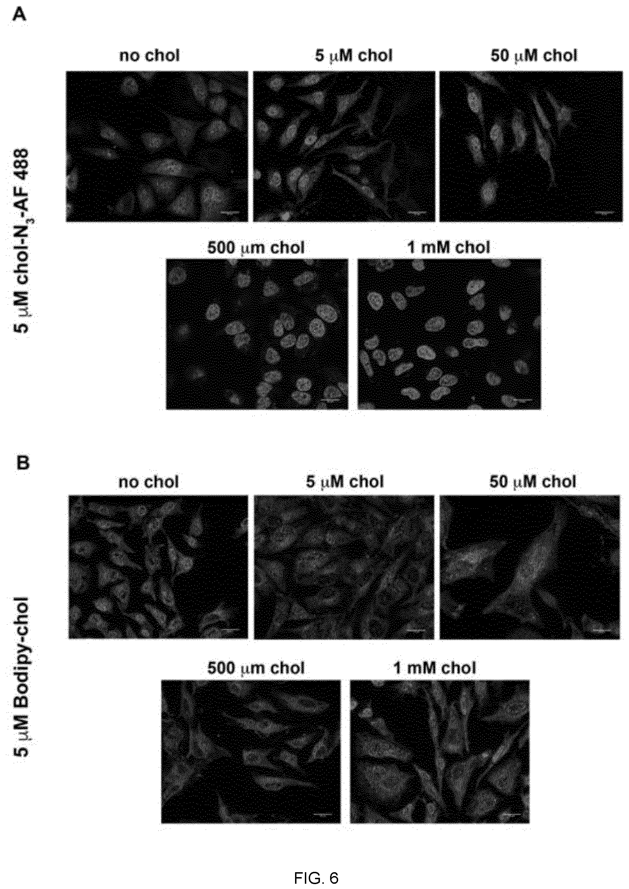

**Figure 6****.** *Competition* assay *to characterize chol analogs uptake in cells.* (A) HeLa cells were co-incubated with a fixed concentration of chol-N₃ prelabeled with AF 488-DBCO (5 µM) and increasing concentration of chol (0-1 mM) in cell culture media. (B) As described in (A) but using Bodipy-chol (5 µM). After incubation, the cells were fixed and stained with DAPI. Representative images of n=3 independent experiments.

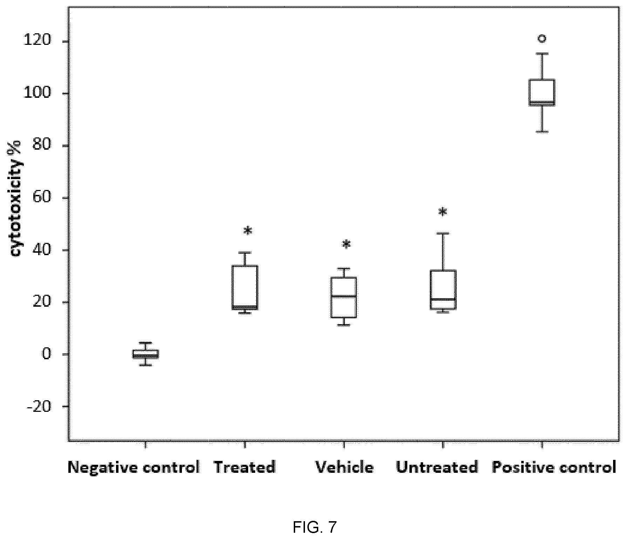

**Figure 7****.** *Cytotoxic effect of chol-N₃ in HeLa cells.* Overnight treatment of HeLa cells with either 25 µM chol-N₃ (Treated) or 0.4% ethanol (Vehicle) or left untreated (Untreated) for 18 h. LDH release into the medium was measured along with negative control (culture medium samples), untreated, and lysed positive control cell samples (lysed with lysis solution). Cytotoxicity is expressed as the percentage of the LDH release of the positive control samples. Data from three independent experiments in quadruplicate are shown. Asterisks denote statistical differences (* = p<0.05) versus both Negative control and Positive control (Kruskal Wallis).

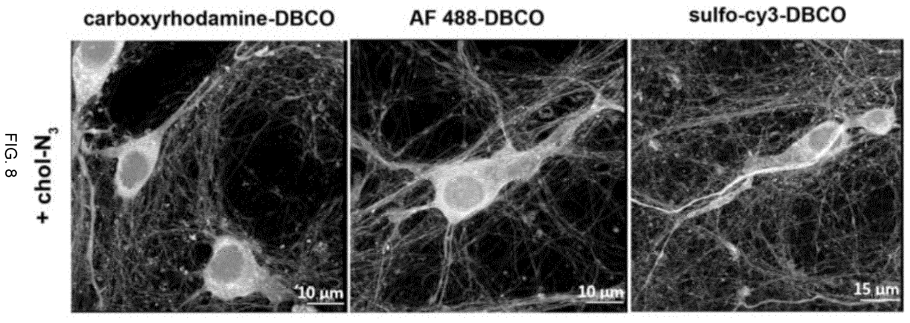

**Figure 8****.** *Chol-N₃ distribution in living neurons labeled with different fluorophores linked to DBCO.* Cortical neurons (DIV14) were treated with 15 µM chol-N₃ for 16 h, washed and then subjected to Cu-free click reaction using different fluorophore dyes linked to DBCO (25 µM), washed out to remove non-bound probe and visualized by confocal microscopy. Representative images of n=3 independent experiments.

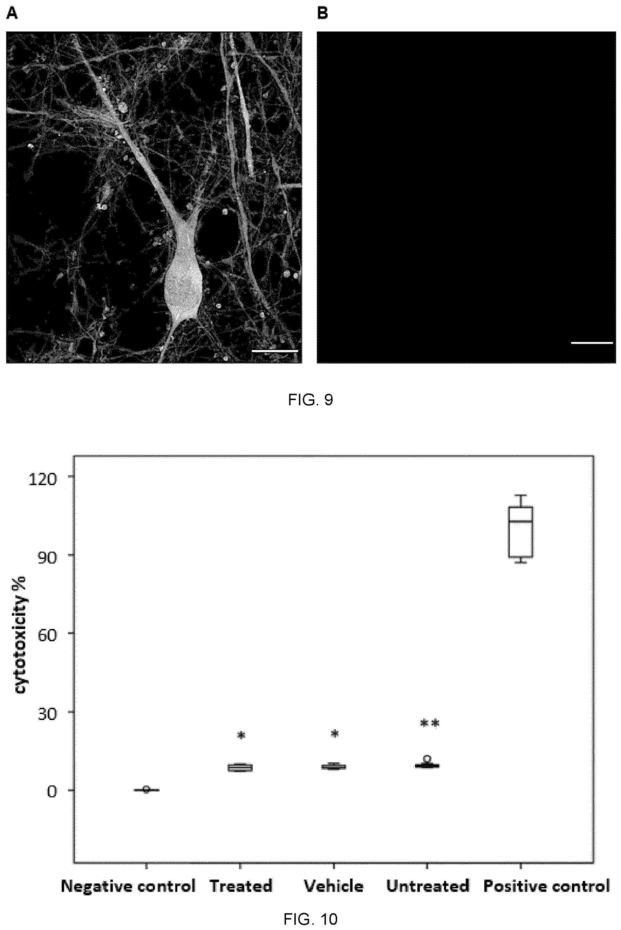

**Figure 9****.** *In vivo chol-N₃ distribution in primary cortical neurons.* A) Neurons were grown in the presence of 15 µM chol-N₃ for 16 h and then subjected to Cu-free click labeling with 25 µM AF 546-DBCO for 30 min at 37°C, washed and cells were imaged. B) Neurons were treated with vehicle (EtOH) for 16h and then treated with 25 µM AF 546-DBCO for 30 min at 37°C, washed and imaged. Scale bar = 10 µM.
**Figure 10****.** *Cytotoxic effect of chol-N₃ in primary neurons.* Overnight treatment of DIV14 rat cortical neurons with either 15 µM chol-N₃ (Treated) or 0.2% ethanol (Vehicle) or left untreated (Untreated) for 18 h. LDH release into the medium was measured along with negative control (culture medium samples), untreated, and lysed positive control cell samples (lysed with lysis solution). Cytotoxicity is expressed as the percentage of the LDH release of the positive control samples. Data from two independent experiments in quadruplicate are shown. Asterisks denote statistical differences (** = p<0.01, *= p<0.05) versus both Negative control and Positive control (Kruskal Wallis).

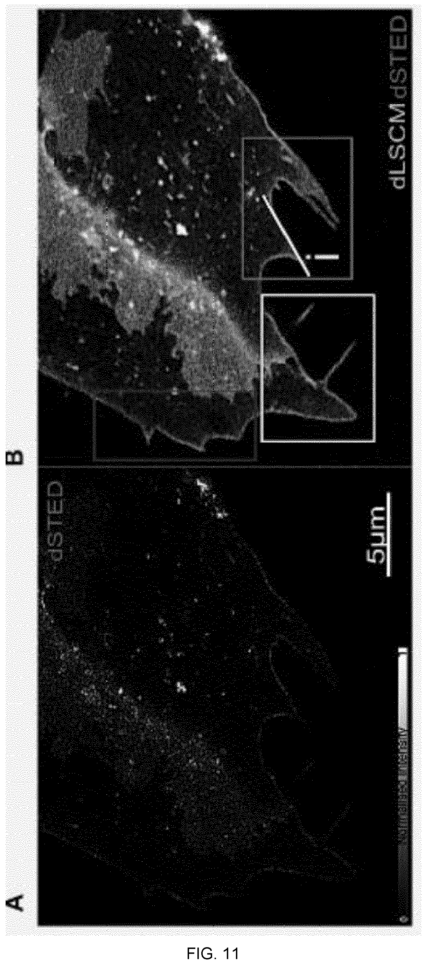

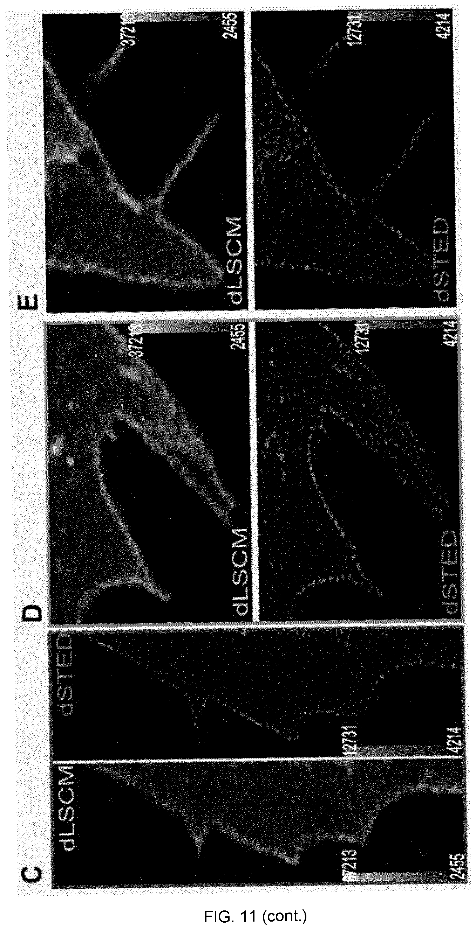

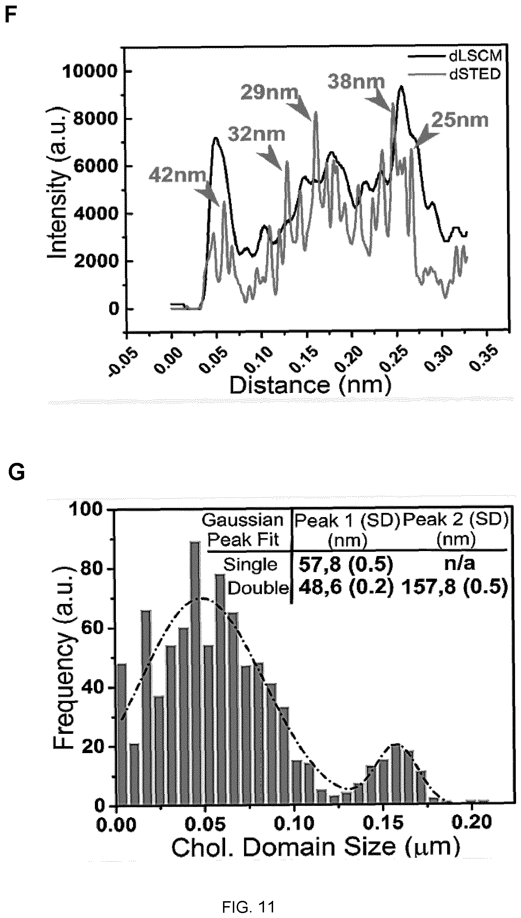

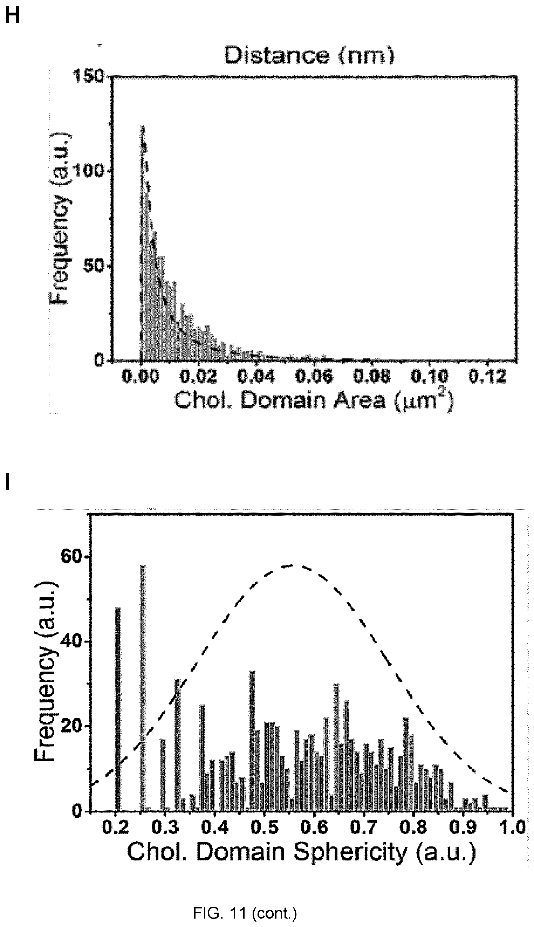

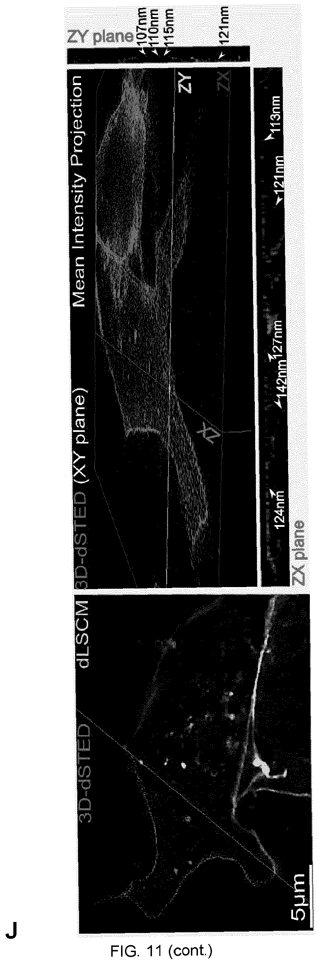

**Figure 11****.** *Cholesterol-rich domains (lipid rafts) distribution and characterization at the nanoscale in living cells.* (A) Overview 2D super-resolution image of chol-N₃ distribution in living SH-SY5Y cells treated with chol-N₃ for 16 h, labeled with AF 488-DBCO and imaged *in vivo* by STED nanoscopy. The image clearly shows punctate chol nanodomains distribution through the PM. (B) Super-imposed image of PM chol-N₃ distribution in living cells visualized either by conventional confocal (LSCM) or STED nanoscopy. (C-E) Closeups images into the marked area (dark grey, white and light grey frames) from B highlighting the improvement in spatial resolution of chol nanodomains within the PM in the STED (dark grey) vs. confocal (white) modes. The color bar indicates the image brightness, and the values at the red and green scale bar, indicate the dynamic range used. (F-I) Nanoscale chol-rich domain characterization across the PM. (F) Representative intensity profile measurement along the solid white line (i) marked in B according to LSCM (dark grey line profile) or STED (light grey profile) and size quantification of selected peaks, using the Full Width at Half Maximum (FWHM) calculation. (G) Chol-rich nanodomains size, (H) area, and (I) shape characterization within the PM. Data were calculated from different PM area. (J) Representative 3D confocal (white) and STED (dark grey) microscopy images of chol-N₃ distribution across the PM of living SH-SY5Y cells (left panel) showing clearly an increase in chol-N₃ signal and resolution by STED over LSCM microscopy. 3D-STED overview image showing the orthogonal view (plane XY, ZX, and ZY). White arrows point out single chol nanoscale domains over the ZY and ZX planes. Corresponding chol-rich domains size quantification is depicted too. Representative images of n=5 independent experiments.

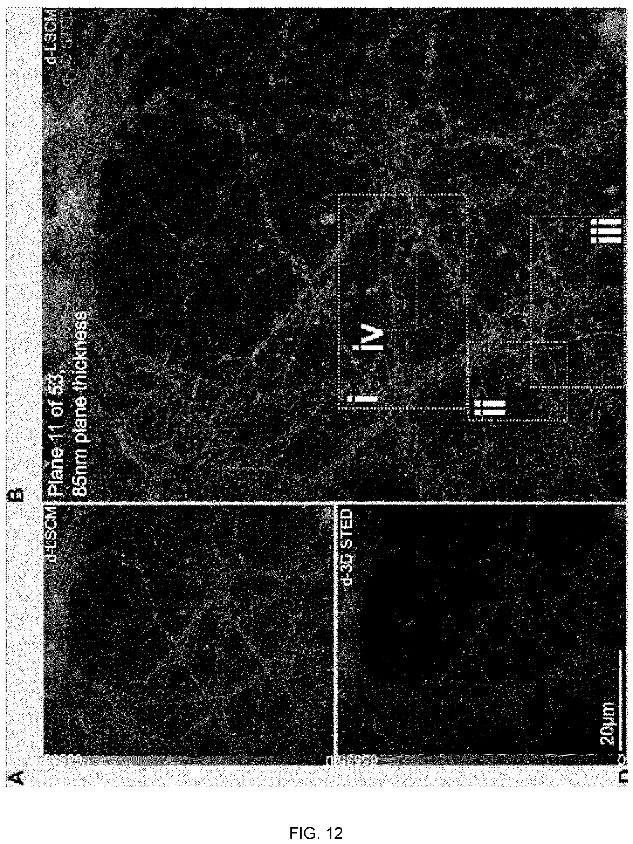

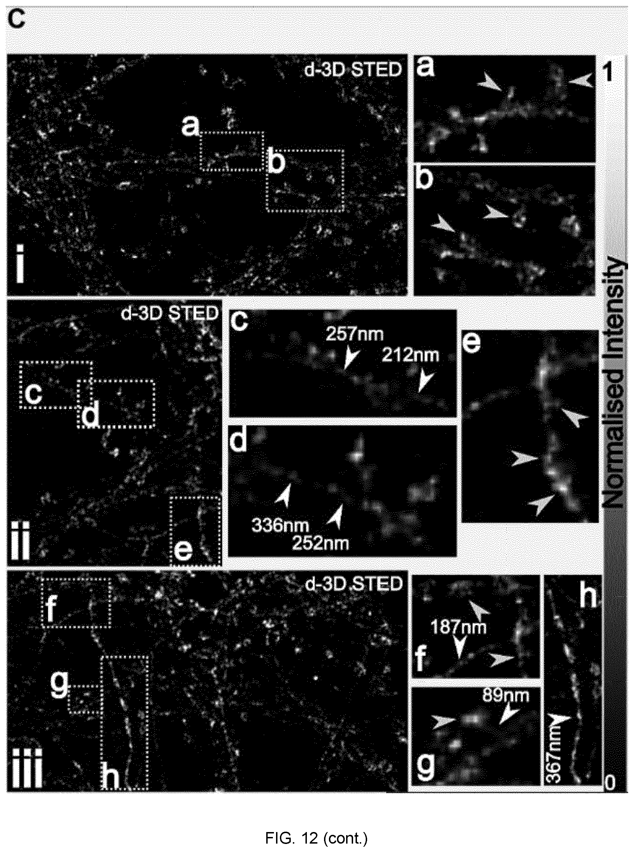

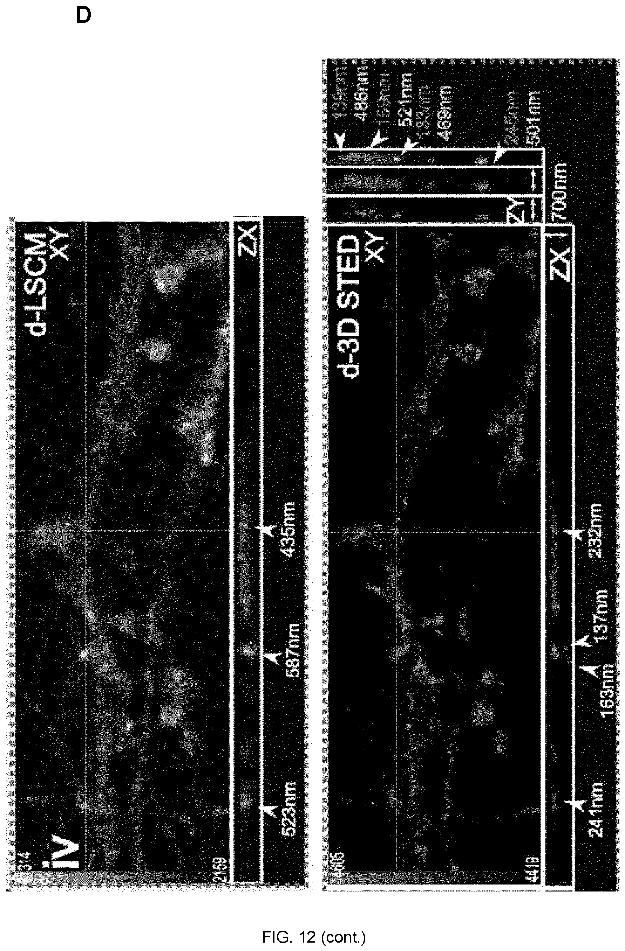

**Figure 12****.** *2D and* *3D* *LSCM and STED microscopy of nanoscale chol-rich domain distribution through cortical neurons.* (A) Cortical neurons treated with chol-N₃ were labeled *in vivo* with AF 488-DBCO, and visualized by conventional LSCM (top white image) and STED nanoscopy (bottom dark grey image). The color bar indicates the image brightness, and the values at the dark grey and white scale bar, indicate the dynamic range used. (B) Super-imposed image of chol-N₃ distribution in living neurons visualized either by conventional confocal (LSCM) or STED nanoscopy showing an increase in chol lateral resolution in STED nanoscopy compared to LSCM. (C) Closeup images into the marked area (inset i-iii) from B and the corresponding magnified white boxes (a-h) for clarity to display nanoscale chol distribution. White arrows point out single chol nanodomains structures and size; white arrows point out chol hot intensive points. (D) 3D LSCM and STED microscopy of the magnified red area from B (inset iv) displaying lateral and axial chol enriched lipid nanodomains distribution in living neurons. The 3D image on the left was obtained using a LSCM, while that on the right was taken using a STED microscope. White arrows in ZX and ZY orthogonal views point out chol nanodomains structures that were highly resolved in STED nanoscopy (dark grey size values) compare to LSCM (white size values). Representative images of n=5 independent experiments.

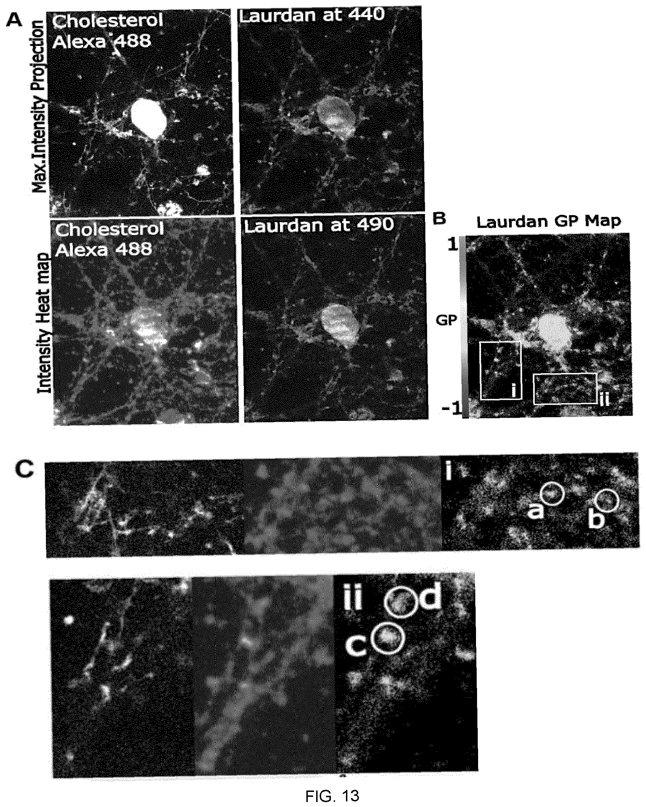

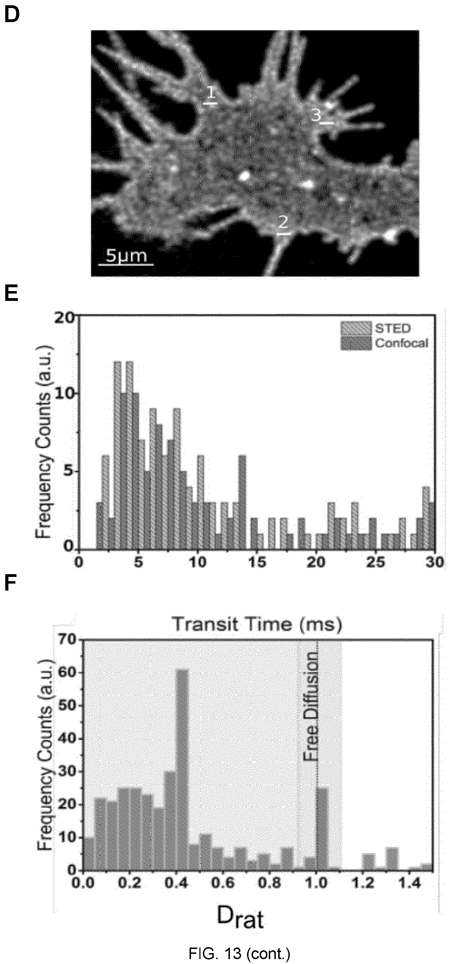

**Figure 13**. *PM chol-rich nanodomains heterogeneity and molecular diffusion analysis in living cells.* (A) Cholesterol-N₃ distribution and Laurdan labeling in neurons. Cortical neurons (DIV14) treated with chol-N₃ were washed and click label with AF 488-DBCO and Laurdan and visualized by confocal microscopy. Overall chol-N₃ distribution (Maximum intensity projection (top left panel), intensity heat map (bottom left panel), and Laurdan raw ratiometric confocal image in the ordered (440 nm top right panel) and disordered (490 nm bottom right panel) channels. (B) Intensity shifts between the ordered and disordered channels from A are quantified as generalized polarization (GP) map, and GP values are plotted as Laurdan GP histogram. (C) Insets I and II from B and the corresponding GP histogram quantification showing chol-N₃ nanostructures trapped in ordered laurdan area (depicted as white circles named b and d in inset i and ii, respectively) and disordered region (depicted as white circles named a and c in inset i and ii, respectively). Representative images of n= 3 independent experiments. (D) Experimental LIESS-FCS recording of chol-N₃ in the plasma membrane of live SH-SY5Y cells. Representative overall confocal image of the plasma membrane indicating homogenous lipid distribution with some rare intense clusters. (E) Frequency histogram of PM chol-N₃ transit time from confocal and STED correlation carpets (7 curves in confocal and STED). (F) Values of Drat (Drat= DSTED/Dconf) for each transit time resulting from the analysis of the correlation carpets from 3 different independent experiments confirming two major defined chol diffusion (trapping and free) modes.

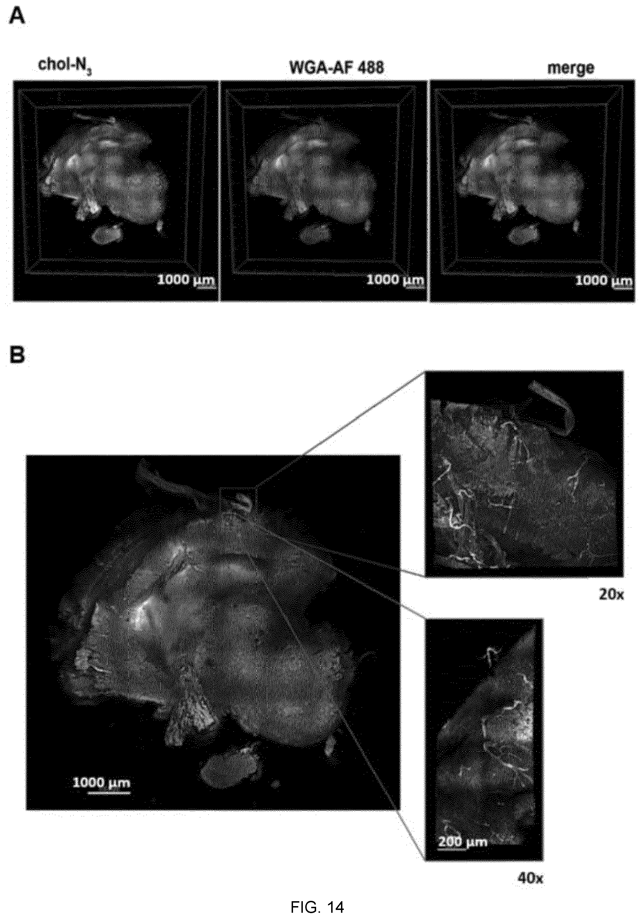

**Figure 14****.** *In depth brain chol distribution.* (A) 3D volumetric reconstruction of rat brain slice (700 µM) labeled ex vivo with chol-N₃ and subjected to cycloaddition reaction with sulfo-cy3-DBCO. PM and nuclei labelling were done using VGA-AF 488 and Hoechst, respectively. (B) 3D volumetric reconstructions of representative cortex sections from B are presented at higher magnifications in image boxes (right). Objectives used for confocal imaging: (A) HC Fluotar 10/0.3, (B) HC Apochromat 20/0.77 (upper inset) and HC Apochromat 40/0.75 (lower inset). Representative images of n=3 independent experiments.

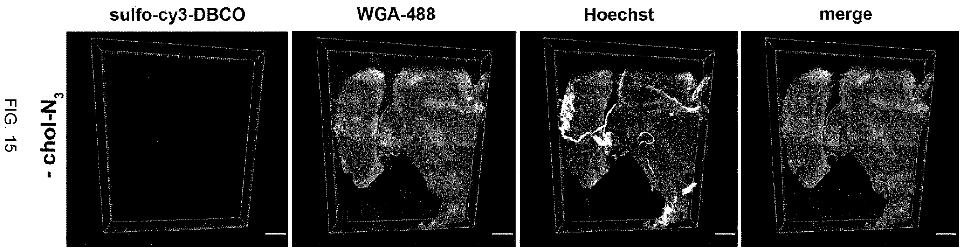

**Figure 15****.** *In situ brain chol-N₃ distribution in depth control.* 3D reconstruction of rat brain slice (700 µM) labeled with chol and subjected to cycloaddition reaction with sulfo-cy3-DBCO. PM and nuclei were labeled with VGA-AF 488 and Hoechst, respectively. Sample was imaged by confocal microscopy. Objective used for confocal imaging: HC Fluotar 10/0.3. Representative images of n=3 independent experiments. Scale bar = 1000 µM.

### DETAILED DESCRIPTION OF THE INVENTION

The present invention relates to the provision of a new chol probe for detection, visualization, imaging or monitoring of cholesterol.

The terms "click chemistry", "click chemistry reaction" and "click reaction" are used interchangeably herein and are intended to be consistent with their use in the art. They refer to the class of reactions that are very efficient and selective and can be used to stitch molecules together in high yields, as originally described by Sharpless and coworkers in 2001. Click chemistry includes reactions such as, but not limited to, azide-alkyne cycloaddition to give the corresponding triazol ring. In the present invention, click chemistry refers to strain-promoted azide-alkyne cycloaddition to form a cycloalkyl-triazolyl group, preferably a cycloocta-triazolyl group.

Unless otherwise defined, all technical terms used herein have the same meaning as commonly understood by one of ordinary skill in the art to which this invention belongs.

All the embodiments and definitions disclosed in the context of one aspect of the invention are also applicable to the other aspects of the invention.

### Method and use for detecting or monitoring cholesterol in cell membranes

In an aspect, the invention relates to the use of a compound of formula (I) 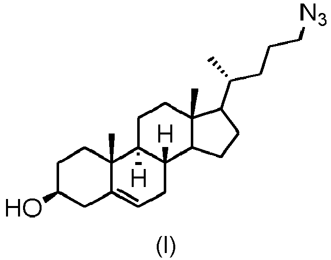 for detecting and/or monitoring cholesterol in cell membranes *in vitro, ex vivo or in vivo,* wherein the compound of formula (I) is used in combination with a cycloalkyne compound labeled with a detectable agent.

In another aspect the invention is directed to an *in vitro, ex vivo* or *in vivo* method for detecting cholesterol in cell membranes comprising:
i) administering to a cell population or to an organism the compound of formula (I) 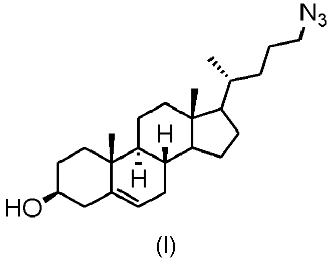 under conditions adequate for the inclusion of said compound in the cell membranes of the cell population or in the cell membranes of the cells of the organism;
ii) contacting the obtained cell population or the cells of the organism of step i) with a cycloalkyne compound labeled with a detectable agent; and
iii) detecting the detectable agent in the desired cell membranes.

The term "*in vitro*", as used herein, refers to the fact that the method is not carried out on the body of a human or animal subject, but rather on cells or fluids isolated from said subject or in a test tube.

The term "ex *vivo"* as used herein, refers to a process, which takes place outside an organism. It refers to experimentation or measurements done in or on tissue from an organism in an external environment with minimal alteration of natural conditions.

The term "*in vivo*" is used herewith, to refer to a process, experimentation or measurement in which the effects of various biological entities are tested on whole, living organisms, usually animals, including humans, as opposed to a tissue extract or dead organism.

In a particular embodiment, the uses and methods of the invention can be performed *in vitro*, *ex vivo* and *in vivo.* In an embodiment, they refer to an *in vitro* or ex *vivo* use and/or method. In another embodiment, it refers to an *in vivo* method.

As used herein the term "detecting" and derivations of this term refer to determining the presence, absence, amount, localization, distribution or organization of an analyte in a sample. In the present case, the analyte is cholesterol and the sample is a cell population or an organism. According to the present invention, "detecting" also includes visualizing, determining, observing, evaluating, quantifying, measuring or imaging cholesterol. This term includes detecting cholesterol at a time point and detecting cholesterol over time (also denoted herein as "monitoring" and derivations of this term).

Therefore, as used herein, the term "monitoring" and derivations of this term refer to the detection of cholesterol over time. It includes visualizing, determining, observing, evaluating, quantifying, measuring and/or imaging cholesterol over time, including its activity or evolution over time, for example to determine whether any changes take place/have taken place. The change may be, for example, the amount or the distribution of cholesterol in the cell membranes, or the diffusion of cholesterol across the cell membranes, or intracellular vesicle trafficking and fusion with the plasma membrane or the membranes of other organelles along time. Detection over time or monitoring includes continued and intermittent detection. In a particular embodiment, monitoring cholesterol may include monitoring cholesterol dynamics, monitoring cholesterol metabolism or monitoring the effect of external stimuli (e.g. temperature, pressure, addition of a product) on the cholesterol distribution, organization or amount.

When the method of the invention is directed to monitoring cholesterol, steps i) and ii) are performed as defined herein and step iii) is performed over a period of time.

As used herein, "cholesterol" includes, without limitation, free or natural cholesterol, esterified cholesterol, i.e., cholesteryl esters, and non-esterified cholesterol, i.e., free-cholesterol.

The present invention takes advantage of the endogenous machinery of living cells to incorporate detectable cholesterol analogs within the cell membranes of a cell population or of the cells of an organism, thus the chemical cholesterol analog is inserted into the cell membranes instead of natural cholesterol. Therefore, within the context of the present invention, the expression "detecting cholesterol" or "monitoring cholesterol" refers to the detection or monitoring of a cholesterol analog. In this sense, as shown in the examples of the present application, the cholesterol analog of the invention retains the biophysical, biochemical and metabolic properties of the natural cholesterol. Therefore, detection or monitorization of the chol analogue of the invention equates to the detection or monitorization of natural chol. Thus, within the context of the present invention, the cholesterol analogue is the compound of formula (I). Said compound can be detected through formation of the corresponding adduct with the cycloalkyne compound labelled with a detectable agent.

The terms "cell membrane" or "membrane of a cell" refer either to the plasma membrane or to the membrane of any of the organelles of a cell. "Plasma membrane" relates to the lipid bilayer that surrounds the protoplasm of a cell. This membrane is not to be confused with the cell wall that covers plant and yeast cells. "Organelles" refer to specialized subunits within a cell that has a specific function. Organelles may be enclosed within lipid bilayers or single membrane compartments and include endoplasmic reticulum, Golgi apparatus, mitochondria, nucleus and vacuoles.

"Administering" refers to delivering a foreign substance or a precursor thereof to one or more cells, such as a tissue or organism, for example a mouse or a human. Means of administering the foreign substance vary depending on the cell's environment. For example, a foreign substance can be delivered to a cell in culture by adding the substance to the cell culture media. In a particular embodiment, the compound of formula (I) is administered to a cell culture or to an extracted tissue. In another particular embodiment, the compound of formula (I) is administered to an organism. Delivery of a foreign substance to an organism, a cell in a body organ or tissue might require more sophisticated means of delivery, including, but not limited to, implantation, direct injection, injection into the bloodstream or lymphatic system, encapsulated or unencapsulated oral delivery, foodstuffs, solutions, gels, ointments, and the like.

In a particular embodiment, administering means that the compounds of the present invention are introduced into a sample having at least one cell, or population of cells, in a test tube, flask, tissue culture, plate or the like and incubated at a temperature and time sufficient to permit uptake of the compounds of the invention into the cell membranes.

In a particular embodiment, the compound of formula (I) is administered as such, either directly or as a composition comprising it. That is, it is not part of a more complex structure.

In another embodiment, the compound of formula (I) is administered within a liposome or nanoparticle, wherein the compound of formula (I) may be incorporated into the lipid bilayer of the liposome or into the nanoparticle. Preferably, when is intended to reach the brain, then the compound of formula (I) is administered within a liposome or a nanoparticle.

In a particular embodiment, the compound of formula (I) is administered within a liposome.

"Liposomes" are self-assembling structures comprising one or more lipid bilayers, each of which comprises two monolayers containing amphipathic lipid molecules oppositely oriented. Amphipathic lipids comprise a polar (hydrophilic) headgroup region covalently linked to one or two non-polar (hydrophobic) acyl chains. Energetically unfavorable contacts between the hydrophobic acyl chains and the surrounding aqueous medium induce the amphipathic lipid molecules to arrange themselves such that their polar headgroups are oriented towards the bilayer's surface, while the acyl chains reorient towards the interior of the bilayer. An energetically stable structure is thus formed in which the acyl chains are effectively shielded from coming into contact with the aqueous environment. Liposomes can have a single lipid bilayer (unilamellar liposomes, "ULVs"), or multiple lipid bilayers (multilamellar liposomes, "MLVs" or "SPLVs"). Each bilayer surrounds, or encapsulates, an aqueous compartment.

Liposomes can have a variety of sizes, e.g., an average diameter as low as 25 nm or as high as 10,000 nm or more. Size is affected by a number of factors, e.g., lipid composition and method of preparation, well within the purview of ordinarily skilled artisans to determine and account for, and is determined by a number of techniques, such as quasi-elastic light scattering, also within the artisans' purview.

Various methodologies, also well within the purview of ordinarily skilled artisans, such as sonication, homogenization, French Press application and milling can be used to prepare liposomes of a smaller size from larger liposomes. Extrusion (see, e.g., U.S. Pat. No. 5,008,050) can be used to size reduce liposomes, that is to produce liposomes having a predetermined mean size by forcing the liposomes, under pressure, through filter pores of a defined, selected size. Tangential flow filtration (WO89/008846), can also be used to regularize the size of liposomes, that is, to produce a population of liposomes having less size heterogeneity, and a more homogeneous, defined size distribution.

Liposomes of this invention can be unilamellar, oligolamellar or multilamellar.

Liposomes suitable for the administration of the compound of formula (I) can comprise other lipids typically employed as liposome constituents, in addition to the compound of formula (I).

Liposomes can be composed of a variety of lipids, both amphipathic and nonamphipathic, obtained from a variety of sources, both natural and synthetic. Suitable liposomal lipids include, without limitation, phospholipids such as phosphatidylcholines ("PC's", such as DDPC, DLPC, DMPC, DPPC, DSPC, DOPC, POPC, DEPC, DLOPC, EPC, HEPC, HSPC, MSPC, PMPC, PSPC, SMPC, SOPC, SPPC), phosphatidylethanolamines ("PE's"; such as DEPE, DELPE, DMPE, DPPE, DSPE, DOPE), phosphatidylserines ("PS's", such as DLPS, DMPS, DPPS, DSPS, DOPS), phosphatidylglycerols ("PG's", such as DEPG, DLPG, DMPG, DPPG, DSPG, DOPG, POPG), phosphatidylinositols ("PI's") and phosphatidic acids ("PA's", such as DMPA, DPPA, DSPA, DEPA, DLPA, DOPA). Such phospholipids generally have two acyl chains, these being either both saturated, both unsaturated or one saturated and one unsaturated; said chains include, without limitation: myristate, palmitate, stearate, oleate, linoleate, linolenate, arachidate, arachidonate, behenate and lignocerate chains. Additional non-phosphorous containing lipids that can become incorporated into liposomes include stearylamine, dodecylamine, hexadecylamine, isopropyl myristate, triethanolamine-lauryl sulfate, alkyl-aryl sulfate, acetyl palmitate, glycerol ricinoleate, hexadecyl stereate, amphoteric acrylic polymers, polyethyloxylated fatty acid amides, DDAB, dioctadecyl dimethyl ammonium chloride (DODAC), 1,2-dimyristoyl-3-trimethylammonium propane (DMTAP), DOTAP, DOTMA, DC-Chol.

In a particular embodiment, the compound of formula (I) is administered in a liposome comprising at least a phospholipid as defined above, preferably a phosphatidylethanolamine, such as DOPE.

The compound of formula (I) can be also administered in a liposome comprising at least a phospholipid and at least a non-phosphorous containing lipid.

In a preferred embodiment, the liposomes for the delivery of the compound of formula (I) to the cell population are fusogenic liposomes. A "fusogenic" liposome as defined herein is a liposome that is capable of interacting with a cell membrane in a way that permits the contents of the liposome to enter the cytoplasm of the cell. Without limitation, this interaction can take the form of complete or partial fusion of the membranes, or adjacent, contemporaneous and localized disruptions of the cellular and liposomal membranes that allows passage of the liposome contents into the cytoplasm of the cell. In a particular embodiment, once the fusogenic liposome has delivered the compound of formula (i) in the cytoplasm of the cell, the compound of formula (i) is transported to the cell membranes by the endocytic machinery of the cell.

In another embodiment, the compound of formula (I) is administered within a nanoparticle. The term "nanoparticle", in the context of the present invention, refers to colloidal systems of the spherical type, rod type, polyhedron type, etc., or similar shapes, having a size less than 1 micrometer (µm), which are individually found or are found forming organized structures (dimers, trimers, tetrahedrons, etc.), dispersed in a fluid (aqueous solution). In a particular embodiment, the nanoparticles suitable for putting the invention into practice have a size less than 1 µm, generally comprised between 1 and 999 nanometers (nm), typically between 5 and 500 nm. The mean particle diameter is the maximum mean particle dimension, with the understanding that the particles are not necessarily spherical. The shape of said nanoparticles can widely vary; advantageously, said nanoparticles will adopt any optically efficient shape such as spheres, rods, stars, cubes, polyhedrons or any other variant as well as complex associations of several particles; in a particular embodiment, the shape of the nanoparticles for putting the invention into practice is spherical or substantially spherical. The shape can be suitably evaluated by conventional light or by means of electron microscopy techniques.

Nanoparticles suitable for use in the present invention include polymeric nanoparticles, lipid nanoparticles and metal nanoparticles.

By "cell population" is meant an *in vitro* or ex *vivo* collection of cells. Thus, the cell population may be referred to as an *"in vitro* or *ex vivo* cell population". It may optionally comprise extracellular compounds and/or extracellular medium. Any reference herein to the analysis of a "cell population" should be understood to mean that the entire cell population, or a sample thereof, might be analyzed.

In an embodiment, the cell population is a population of living cells at least in step i) of the method of the invention; preferably at least in steps i) and ii) of the method of the invention. In a further embodiment, the cell population is a population of living cells throughout the method or use of the invention.

In another embodiment, the cells in the cell population are fixed before step iii) in the method of the invention. In another embodiment, the cells in the cell population are fixed before step ii) in the method of the invention.

The cell population is an animal cell population. More preferably, it is a mammalian cell population. Non limiting examples of mammalian organisms from which the cell population may be obtained include humans, monkeys, cattle, guinea pig, horse, ferret, fowl, alpaca, goats, cats, dogs, rabbit, buffalo pigs, rats, mice, rabbits or hamsters or another vertebrate species. It may originate from a livestock, domestic or laboratory animal. In a particular embodiment the cell population is human.

Unless otherwise specified herein, any reference herein to a "cell" should be understood to be a reference to a cell that is part of a cell population.

Optionally, the cell population may comprise or consist of adult, embryonic, and/or foetal cells, e.g., human embryonic cells or human adult cells.

Optionally, the cell population may comprise or consist of stem cells and/or differentiated cells. Optionally, stem cells may be totipotent stem cells, pluripotent stem cells, multipotent stem cells, and/or oligopotent stem cells.

Optionally, the cell population may comprise or consist of cells originating from, or having the characteristics of, cells of adrenal gland tissue, appendix tissue, bladder tissue, bone, bowel tissue, brain tissue, breast tissue, bronchi, ear tissue, oesophagus tissue, eye tissue, endometrioid tissue, gall bladder tissue, genital tissue, heart tissue, hypothalamus tissue, kidney tissue, large intestine tissue, intestinal tissue, larynx tissue, liver tissue, lung tissue, lymph nodes, mouth tissue, nose tissue, pancreatic tissue, parathyroid gland tissue, pituitary gland tissue, prostate tissue, rectal tissue, salivary gland tissue, skeletal muscle tissue, skin tissue, small intestine tissue, spinal cord, spleen tissue, stomach tissue, thymus gland tissue, trachea tissue, thyroid tissue, ureter tissue, urethra tissue, soft and/or connective tissue, peritoneal tissue, blood vessel tissue and/or fat tissue.

The term cell population includes a primary cell culture, a secondary cell culture, a cell line, a tissue, an organ and/or an organoid.

A "primary cell culture" is a culture of cells that were dissociated from the parental tissue using mechanical or enzymatic methods. A primary cell culture may, e.g., be an adherent cell culture or a cell suspension culture.

In an embodiment, the cells are a primary culture of cortical or hippocampal neurons. In another embodiment, the cells are cholesterol-enriched primary cells. Cholesterol-enriched cells can be obtained incubating the cells with cholesterol or a cholesterol derivative, for example with the compound of formula (I), preferably in ethanol for a time between 6 and 18 hours or incubating cells with purified lipoproteins LDL.

In another particular embodiment, the cells are cholesterol-enriched primary cultures of rat cortical or hippocampal neurons.

A "secondary cell culture" is a culture of cells which may e.g., be derived from a primary cell culture via multiple cell passages.

A "cell line" is a cell population which is typically uniform and which can be cultured *in vitro* for several passages. A cell line may, e.g., be derived from a secondary cell culture, from a tumour, and/or from an embryo. A cell line may optionally be "immortalized", in which case it can be cultured indefinitely. Immortalized cell lines typically have one or more mutations that override the cells' natural growth controls. Optionally, the cell population may comprise or consist of one or more of the following cell lines: Hela (cervical adenocarcinoma), SH-SY5Y, HCN-2, CHLA-03-AA, C8-D1A, Hs 683, CHLA-02-ATRT, HCM-BROD-0214-C71, SK-N-SH, PFSK-1, IMR-32, CHP-212, C8-S, CCF-STTG1, 3T3, Chinese hamster ovary (CHO), BHK, HEK293 (embryonic kidney cells), SKNBE2 (neuroblastoma), SW480 (colon carcinoma), PC3M, HOP62, T24, MES_SA (uterine sarcoma) and/or HepG2.

The cell population may comprise or consist of one or more different cell types, in which case it may refer to tissues or organs.

By "tissue" is meant a collection of cells having a similar morphology and function.

"Organ" as used herein, refers to a differentiated part of an organism which with a specific function. An organ includes, but is not limited to, bladder, brain, nervous tissue, glial tissue, esophagus, fallopian tube, heart, pancreas, intestines, gallbladder, kidney, liver, lung, ovaries, prostate, spinal cord, spleen, stomach, testes, thymus, thyroid, trachea, urogenital tract, ureter, urethra, uterus, breast, skeletal muscle, skin, bone, and cartilage.

Optionally, the cell population may be mutant and/or transgenic. In addition, the cell population may have been obtained by CRISPR Cas9 technology.

In a preferred embodiment, the cell population derives from brain tissue.

As mentioned above, the cell population may optionally be an organoid.

By an "organoid" or "organoid tissue" is meant an organized tissue formed *in vitro* or *ex vivo,* said tissue having a cellular organization and gross morphology similar to that of the same tissue type *in vivo,* for at least a subset of the cells of the tissue. Unlike classical two-dimensional cell cultures, organoid systems permit 3D cell growth, cell movement, cell differentiation and more physiologically representative cell-cell interactions. In contrast to traditional 2D cell cultures, organoids share similar physical, molecular and physiological properties with the *in vivo* tissue of the same type. Organoids therefore represent effective models for understanding organ development, tissue morphogenesis, and/or the genetic or molecular basis of diseases.

Organotypic tissue may be generated from organ "explants", i.e. cells, or pieces of tissue, that were isolated from primary mammalian organs, or tumour explants, i.e. cells or pieces of tissue isolated from a tumour. Explants may, e.g., be obtained from resected tissue and/or biopsies. Organotypic tissue may alternatively be generated by expanding ES cells, iPS cells or primary stem cells that have been purified from organs.

It is within the competencies of one of ordinary skill in the art to provide an organoid for their particular purposes. Methods for the production of organoids are well-known in the art.

An "organoid", as used herein, may optionally include a scaffold, which is a preformed solid support that imparts or provides short-term (hours to two weeks in culture) structure or support to the tissue or is required to form the tissue.

By "at least a subset of cells" is meant at least two cells, e.g., at least 10%, 15%, 20%, 25%, 30%, 35%, 40%, or 50% of the cells of the tissue. Optionally, substantially all of the cells within the organoid have a cellular organization and gross morphology similar to that of the same tissue type *in vivo.* By "substantially all of the cells" is meant at least 90, 91, 92, 93, 94, 95, 96, 97, 98, or 99% of the cells.

By "three-dimensional" is meant an organized tissue having x, y and z axes wherein x and y of the axes are at least 2 mm with z at least 0.05 mm thick, and wherein 1, 2 or all of the axes are as great as 20 cm. Optionally, a three-dimensional tissue may be transferred between in vivo and in vitro environments one or more times, e.g., exactly or at least 2, 3, 4, 5, 6, 7, 8, 9 or 10 times without undergoing any substantial structural and/or shape changes.

The organoid may be any type of organoid, e.g., an organoid of any of the cell and/or tissue types mentioned elsewhere herein. Optionally, the organoid may be selected from, e.g., cerebral organoids, thyroid organoids, intestinal organoids (e.g., small intestinal, colonic, rectum, duodenum or ileum organoids), testicular organoids, hepatic organoids, pancreatic organoids, gastric organoids, epithelial organoids, lung organoids, kidney organoids, muscle organoids, prostate organoids, breast organoids, blood vessel organoids, lymphatic vessel organoids and/or retinal organoids.

Optionally, the organoid may be prepared from terminally differentiated cells of the desired organ, e.g., a "cerebral organoid" may be derived from terminally differentiated brain cells. However, organoids may also be produced from stem cells.

Optionally, the organoid may be prepared from an explant or cells from a patient. The term "similar to that of the same tissue type in vivo" means that the organoid has genetic and phenotypic characteristics that allow it to be recognized by the skilled person as being from or associated with a particular tissue type (such as the tissues listed above). It does not mean that the organoid necessarily has to be genetically and phenotypically identical to the corresponding in vivo tissue type.

Optionally, the organoid may be a mammalian organoid, i.e. it may be derived from cells or an explant taken from a mammal. The mammal may be any mammal of interest, e.g., selected from the mammals listed elsewhere herein. Optionally, the organoid may be a non-human organoid. Optionally, the organoid may be a human organoid.

Optionally, all of the cells within the organoid are differentiated, optionally terminally differentiated. By "differentiated" is meant cells with numerous mature-like characteristics, either chemical or physical. By "terminally differentiated" is meant is not capable of further proliferation or differentiation into another cell or tissue type.

An organoid may comprise a single cell type. More typically, an organoid may comprise a mixture of different cell types.

Optionally, the cell population may comprise or consist of healthy and/or diseased cells.

By "organism" is meant any individual entity that embodies the properties of life. The organism may be a human or non-human. Optionally, it may be mammalian, e.g., originate from a livestock, domestic or laboratory animal. Optionally, it may be murine, guinea pig, hamster, rat, goat, pig, cat, dog, sheep, rabbit, cow, horse, alpaca, ferret, fowl, buffalo, and/or monkey. In a preferred embodiment the organism is a human.

The "adequate conditions" are those known by the person skilled in the art that allows the inclusion of the compound of the invention in the cell membrane, such as temperature and time sufficient to permit uptake of the compounds of the invention into the cell membrane.

In a particular embodiment, when the compound of formula (I) is administered to a cell population, these conditions may include the composition in which the compound of formula (I) is administered on the cell population or the amount of compound. Also, after the administration of the compound of formula (I) to the cell population, the compound is maintained together with the cell population for a period of time, that is incubated, thus, the adequate conditions may also include the incubation time and the incubation temperature.

In an embodiment, the compound of formula (I) is administered as a solution, for example in an organic solvent or in an isotonic solution or a buffer solution. Non-limiting examples of buffers suitable for allowing the inclusion of the compound of the invention into cell membranes include without limitation PBS, TBS, phosphate buffer, HEPES buffer, and citrate buffer. Non-limiting examples of organic solvents include methanol, ethanol or DMSO. Preferably, when the compound of formula (I) is administered in a liposome or a nanoparticle, it is administered in an isotonic solution or buffer solution. Alternatively, when the compound of formula (I) is administered as such, it is preferably administered in an organic solvent.

The amount of the compound of formula (I) will depend both on the size of the sample and on the amount of cholesterol present therein, and it could be readily determined by optimization methods commonly used in the art. By way of indication, the compound concentration in the administered solution may be at least 1 fM, at least 10 fM, at least 100 fM, at least 1 pM, at least 10 pM, at least 100 pM, at least 1 nM, at least 10 nM, at least 100 nM, at least 1 µM, at least 10 µM or more. In an embodiment, the compound of formula (I) concentration is between 0.1 nM and 1 mM, preferably between 0.1 µM and 100 µM.

In a particular embodiment, when the compound of formula (i) is administered ex vivo in cerebral tissue, the compound concentration in the administered solution is preferably between 0.1 mM and 1 mM. In another embodiment, the compound of formula (I) is administered to an organism. In an embodiment, when the compound of formula (i) is administered to an organism, and more particularly, when it is intended that the compound of formula (I) reaches the brain or the organism, the compound is administered within a liposome or nanoparticle at a concentration between 0.1 and 1 mmol/Kg of the compound within the liposome or nanoparticle, more preferably between 0.2 and 0.7 mmol/Kg.

In step (i), the compound of formula (I) can be incubated with the cell population at a suitable temperature and for a time sufficient for allowing the inclusion of the compound of the invention in the plasma membrane of the cell population. The temperature is preferably between 5°C and 50°C, more preferably between 10°C and 50°C, even more preferably between 20°C and 42°C. In a more preferred embodiment, the temperature is between 30°C and 40°C, such as at 37°C. In a particular embodiment, when the method is performed in an organism, the temperature is preferably between 36°C and 41°C; more preferably between 36°C and 38°C. By way of indication, the compound of formula (I) may be incubated with the cell population for at least 30 min, at least 1 h, at least 2 h, at least 3 h, at least 5 h, at least 10 h or more. In an embodiment, the compound of formula (I) is incubated with the cell population between 1 h and 50 h, preferably between 2 h and 40 h, more preferably between 5 h and 30 h.

This step (i) may be carried out at a neutral pH, a pH of 5.5 to 8.5, preferably a pH of 6 to 8, more preferably a pH of 6.5 to 7.5.

In a particular embodiment, the method for detecting cholesterol of the invention further comprises a step of rinsing or washing with a buffer solution (e.g. PBS, TBS, phosphate buffer, citrate buffer) after step (i) and/or (ii) in order to remove excess or non-reacted compound (I) and/or labeled cycloalkyne.

In a preferred embodiment, the method of the invention comprises a rinsing or washing step with buffer solution after step (i) and after step (ii).

The washing step can be repeated several times until all the excess or non-included compound of formula (I) is removed and/or until all the excess or non-reacted labeled cycloalkyne is removed.

In a particular embodiment, when the compound of formula (I) is administered to an organism, the adequate conditions are the physiological conditions of the organism to which the compound is administered. In such case, a period of time of at least 1 hour, or more preferably 2 hours, is preferably waited after the administration of the compound of formula (I) to allow the excess of cholesterol analogue not incorporated into the cell membranes of the cells of the organism to be eliminated from the organism.

Once the compound of formula (I) has been incorporated or included into the cell membranes of the cell population or into the cell membranes of the cells of the organism, and the excess optionally washed out or eliminated from the organism, the resulting cell population or cells of the organism are contacted with the labeled cycloalkyne.

Due to the strain of the cycloalkyne ring, it reacts spontaneously with the compound of formula (I) present in the cell membranes without the need of any metal catalyst. Therefore, the method of the invention proceeds in the absence of a metal catalyst, in particular in the absence of a copper catalyst.

The term "contacting", as used herein refers to the process by which the labeled cycloalkyne of the invention comes into contact with the cell population or the cells of the organism and includes any possible method that allows the introduction of the labeled cycloalkyne into the cell population of cells of the organism and therefore, to react with the compound of formula (I) by way of a click chemistry reaction. Thus, upon cell insertion, the labeled cyclooctyne reacts with the azide group of the compound of formula (I) giving rise to a cholesterol probe suitable for in situ detection of cholesterol. The step of contacting the cell population or the cells of the organism with the labeled cycloalkyne may involve its administration to the cell population or the organism in the same way as the administration of the compound of formula (I). Thus, all the particulars of the administration referred in the first step of the method are equally applicable to the second step of the detection method. This include also a washing step to remove the labeled cycloalkyne not bound to the compound of formula (I), or in the case of the contacting with the cells of an organism, to wait for a period of time, typically for at least 30 minutes, preferably for at least 60 minutes, to allow the excess labeled cycloalkyne to be removed from the organism. In an embodiment, the waiting period is between 30 and 240 minutes, preferably between 60 and 180 minutes.

The click chemistry reaction between the compound of formula (I) and the labeled cycloalkyne may be performed under physiological conditions. Physiological conditions may include an isotonic solution or biological buffer such as cell culture medium, PBS, TBS, phosphate buffer, citrate buffer and the like or the own physiological conditions of the organism to which the compound of formula (I) has been administered.

Click chemistry reaction conditions may alternatively or additionally include a temperature ranging from 5°C to 50°C, more preferably between 10°C and 50°C, even more preferably between 20°C and 50°C. In a more preferred embodiment, the temperature is between 30°C and 40°C, such as at 37°C. In a particular embodiment, when the method is performed in an organism, the temperature is preferably between 36°C and 41°C; more preferably between 36°C and 38°C.

Click chemistry reaction conditions may include a neutral pH, a pH of 5.5 to 8.5, preferably a pH of 6 to 8, more preferably a pH of 6.5 to 7.5.

By way of indication, the click chemistry reaction may be conducted for at least 1 minute, at least 2 minutes, at least 5 minutes, at least 10 minutes, at least 15 minutes, at least 20 minutes or more. In an embodiment, the cell population including the compound of formula (I) in the membrane is contacted with the labeled cycloalkyne between 2 minutes and 40 h, preferably between 5 minutes and 25 h, more preferably between 10 minutes and 10 h. In a more preferred embodiment, it is contacted between 15 minutes and 5 h.

The amount of the labeled cycloalkyne will depend both on the size of the sample and on the amount of cholesterol present therein, and it could be readily determined by optimization methods commonly used in the art. Usually, the labeled cycloalkyne is used in an excess amount with respect to the compound of formula (I) to ensure that all the compound of formula (I) included in the membrane reacts with the labeled cycloalkyne. In an embodiment, at least 1.2 molar equivalents, preferably at least 1.5 molar equivalents, of the labeled cycloalkyne are used. That is, at least 1.2 moles, preferably at least 1.5 moles, of the labeled cycloalkyne are used in step (ii) for each mol of compound of formula (I) administered in step (i).

The labeled cycloalkyne may be administered in an isotonic solution or buffer (e.g. PBS, TBS, phosphate buffer, citrate buffer and the like). By way of indication, the concentration in the administered solution may be at least 1 fM, at least 10 fM, at least 100 fM, at least 1 pM, at least 10 pM, at least 100 pM, at least 1 nM, at least 10 nM, at least 100 nM, at least 1 µM, at least 10 µM or more. In an embodiment, the compound of formula (I) concentration is between 1 nM and 1 mM, preferably between 1 µM and 100 µM.

In an embodiment, the method of the invention is carried out under physiological conditions, more preferably at a pH of 6 to 8, and/or at a temperature between 10°C and 50°C, preferably between 20°C and 40°C, such as 37°C. In a particular embodiment, when the method is performed in an organism, the temperature is preferably between 36°C and 41°C; more preferably between 36°C and 38°C.

In a particular embodiment, when the method is performed in a cell population the method of the invention comprises:
i) administering to a cell population the compound of formula (I) 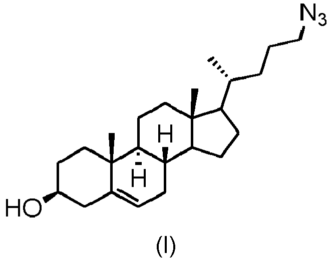 under conditions adequate for the inclusion of said compound in the cell membranes of the cell population;
i') washing with a buffer solution;
ii) contacting the obtained cell population with a cycloalkyne compound labeled with a detectable agent;
ii') washing with a buffer solution;
iii) detecting the presence of the detectable agent in the cell membranes;
   preferably wherein the method is carried out under physiological conditions, more preferably at a pH of 6 to 8, and/or at a temperature between 10°C and 50°C more preferably between 20°C and 40°C.

In a particular embodiment, when the method is performed in an organism, the washing step is replaced by waiting for at least 30 minutes, preferably for at least 60 minutes, to allow the removing of the excess of compound of formula (I) and labeled cycloalkyne from the organism. In an embodiment, the waiting period is between 30 and 240 minutes, preferably between 60 and 180 minutes.

The terms "labeled cycloalkyne" or "cycloalkyne compound labeled with a detectable agent" are interchangeable and refer to a cycloalkyne to which a detectable agent or label has been covalently attached, via an optional linker. Covalent attachment may be performed using a variety of methods and functional group chemistries. Exemplary covalent linkages that may be utilized in the labelling of the cycloalkyne include but are not limited to, amide or ester bond linkages, e.g., via reaction of an amino or hydroxyl group with a carboxylic acid or derivative thereof (e.g., an active ester); carbamate linkages; ether linkages, e.g., via reaction of a hydroxyl group and an electrophilic carbon (e.g., a bromo-alkyl); amino linkage, e.g., via reductive amination, etc..

In some embodiments, the labeled cycloalkyne may be described by the following formula:

Z-L-Y

wherein Z is a cycloalkyne as defined herein; L is an optional linker; and Y is a detectable agent.

The cycloalkyne in the present invention can be any compound known in the art useful in cooper-free click chemistry reactions. As used herein, cycloalkyne includes 7-membered rings (cycloheptynes), 8-membered rings (cyclooctynes), 9-membered rings (cyclononynes) and 10-membered rings (cyclodecynes), where one or two of the C ring atoms may be replaced by N, O, S and/or C=O. Additionally, the cycloalkyne may include functional moieties such as alkyl, cycloalkyl, aryl, heteroaryl, halogens (e.g. fluorines), ethers, alcohols, ketones, esters, acids, amines, amides, maleimides.

In a preferred embodiment, the cycloalkyne is a cyclooctyne. As used herein, the term cyclooctyne includes 8-membered rings where one or two of the C ring atoms may be replaced by N, O, S and/or C=O. Preferably, the cycloalkyne is a cyclooctyne where all the ring atoms are C atoms (cyclooctyne) or an 8-membered ring where one of the ring atoms is a N atom and the other ones being C atoms (azacyclooctyne).

In an embodiment, the cyclooctyne is a cyclooctyne or azacyclooctyne having one or more adjacent aromatic or heteroaromatic rings, having one or more fluorine substitutions, having one or more oxygenated (alkoxy) substutitions, or having one or more C₃-C₇ cycloalkyl ring fusions (e.g. cyclopropyl or cyclobutyl) that cause strain on the cyclooctyne or azacyclooctyne ring, and which may include functional moieties such as alkyl, cycloalkyl, aryl, heteroaryl, halogens, ethers, alcohols, ketones, esters, acids, amines, amides, maleimides. These strained cyclooctynes are known in the art.

In an embodiment, the cyclooctyne is selected from mono- and dibenzo cyclooctyne or azacyclooctyne, mono- and difluoro cyclooctyne or azacyclooctyne, mono- and dialkoxy cyclooctyne or azacyclooctyne, cyclopropyl cyclooctyne or azacyclooctyne, and pirrolo cyclooctyne or azacyclooctyne, which may include functional moieties such as alcohols, ketones, esters, acids, amines, amides, maleimides.

In a further embodiment, the cyclooctyne may be selected from the following compounds:

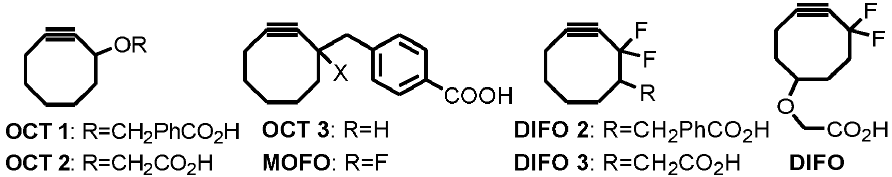

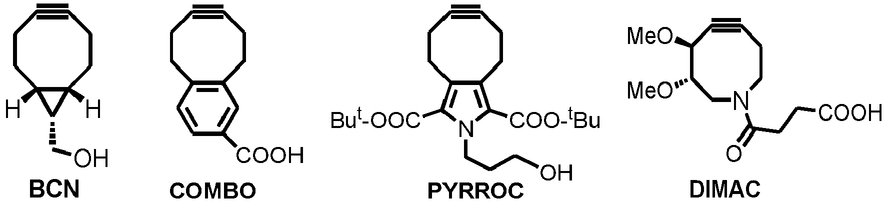

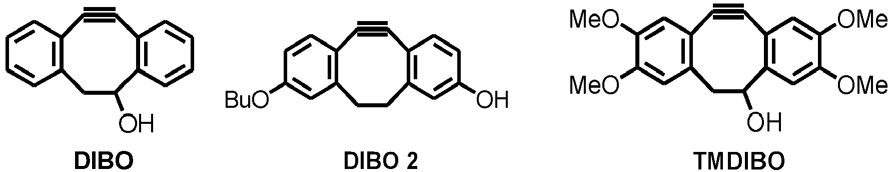

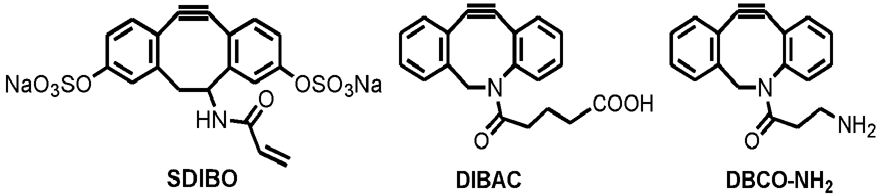

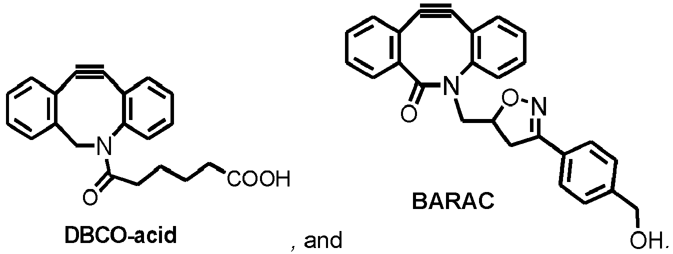

In an embodiment, the cyclooctyne is selected from dibenzo cyclooctyne and dibenzo azacyclooctyne, which may include functional moieties such as alcohols, ketones, esters, acids, amines, amides, maleimides. In a further embodiment, the cyclooctyne group is selected from:

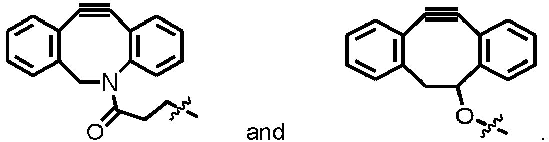

The detectable agent may be covalently attached to the cycloalkyne via a direct bond or via a linker.

In an embodiment, the detectable agent is linked to the cycloalkyne by a linker. As used herein, the term "linker" refers to the molecular fragment or moiety that joints the cycloalkyne and the detectable agent.

In an embodiment, the linker may have a length from 1 to 50 atoms, preferably from 1 to 20 atoms. The linker may comprise an aliphatic, cycloaliphatic, aromatic, heteroaromatic, alkene, alkyne and/or polyethylene groups.

In an embodiment, the linker is a hydrocarbyl chain (e.g. C₁-C₅₀ alkylene, C₂-C₅₀ alkenylene) optionally interspersed with one or more groups selected from -O-, -S-, - NR'-, -CO-, -COO- and -CONR'-, wherein R' is independently selected from H and C₁-C₆ alkyl; and wherein the hydrocarbyl chain may be optionally substituted with one or more substituent groups. The optional substituent groups may be -OR", -OCOR", - COOR", -NR"R"', -NR"-COR"', -CONR"-R"', -COR", -CN, halogen, or a combination thereof, wherein R' and R" are independently H or C₁-C₆ alkyl.

The term "detectable agent", "label", "imaging agent" and "contrast agent", are used herein interchangeably and refer to biocompatible compounds with capacity to be detected either directly or indirectly, the use of which facilitates the differentiation of different parts of the image, by increasing the "contrast" between those different regions of the image. They refer to an atom, molecule, compound or other substance that is useful in diagnosing, detecting or visualizing the uptake of the compounds of the invention through the click-chemistry reactions occurring on the cell membrane.

According to the embodiments described herein, detectable labels may include, but are not limited to, radioactive substances (e.g., radioisotopes, radionuclides, radiolabels or radiotracers), dyes, contrast agents, fluorescent compounds or moieties, luminescent compounds or moieties, enzymes and enhancing agents (e.g., paramagnetic ions).

In an embodiment, the detectable label is a radioactive substance, preferably a radioisotope. Radioactive substances that may be used as detectable labels in accordance with the embodiments of the disclosure include, but are not limited to, ¹⁸F, ³²P, ³³P, ⁴⁵Ti, ⁴⁷Sc, ⁵²Fe, ⁵⁹Fe, ⁶²Cu, ⁶⁴Cu, ⁶⁷Cu, ⁶⁷Ga, ⁶⁸Ga, ⁷⁵Sc, ⁷⁷As, ⁸⁶Y, ⁹⁰Y. ⁸⁹Sr, ⁸⁹Zr, ⁹⁴Tc, ⁹⁴Tc, ⁹⁹mTc, ⁹⁹Mo, ¹⁰⁵Pd, ¹⁰⁵Rh, ¹¹¹Ag, ¹¹¹In, ¹²³I, ¹²⁴I, ¹²⁵I, ¹³¹I, ¹⁴²Pr, ¹⁴³Pr, ¹⁴⁹Pm, ¹⁵³Sm, ¹⁵⁴-¹⁵⁸¹Gd, ¹⁶¹Tb, ¹⁶⁶Dy, ¹⁶⁶Ho, ¹⁶⁹Er, ¹⁷⁵Lu, ¹⁷⁷Lu, ¹⁸⁶Re, ¹⁸⁸Re, ¹⁸⁹Re, ¹⁹⁴Ir. ¹⁹⁸Au, ¹⁹⁹Au, ²¹¹At, ²¹¹Pb, ²¹²Bi, ²¹²Pb, ²¹³Bi, ²²³Ra and ²²⁵Ac. Paramagnetic ions that may be used as detectable labels in accordance with the embodiments of the disclosure include, but are not limited to, ions of transition and lanthanide metals (e.g. metals having atomic numbers of 6 to 9, 21 -29, 42, 43, 44, or 57-71). These metals include ions of Cr, V, Mn, Fe, Co, Ni, Cu, La, Ce, Pr, Nd, Pm, Sm, Eu, Gd, Tb, Dy, Ho, Er, Tm, Yb and Lu. In a more preferred embodiment, the radioactive substance or radioisotope is ⁸⁹Zr.

When the detectable label is a radioactive metal or paramagnetic ion, the label may be reacted with a reagent having a long tail with one or more chelating groups attached to the long tail for binding these ions. In that case, the chelating groups may be covalently attached to the cycloalkyne. The long tail may be a polymer such as a polylysine, polysaccharide, or other derivatized or derivatizable chain having pendant groups to which may be bound to a chelating group for binding the ions. Examples of chelating groups that may be used according to the disclosure include, but are not limited to, ethylenediaminetetraacetic acid (EDTA), diethylenetriaminepentaacetic acid (DTPA), DOTA, NOTA, NETA, porphyrins, polyamines, crown ethers, bis-thiosemicarbazones, polyoximes, DFO (deferoxamin-maleimide) and like groups. The same chelates, when complexed with non-radioactive metals, such as manganese, iron and gadolinium are useful for MRI. Macrocyclic chelates such as NOTA, DOTA, and TETA are of use with a variety of metals and radiometals including, but not limited to, radionuclides of gallium, yttrium and copper, respectively. Other ring-type chelates such as macrocyclic polyethers, which are of interest for stably binding nuclides, such as 223Ra for RAIT may be used. In certain embodiments, chelating moieties may be used to attach a PET imaging agent, such as an AI-18F complex, or DFO-89Zr to a targeting molecule for use in PET analysis. In a preferred embodiment, the chelating group is DFO. In a more preferred embodiment, the conjugate is Omomyc-DFO-89Zr.

Enzymes that may be used as imaging agents in accordance with the embodiments of the disclosure include, but are not limited to, horseradish peroxidase, alkaline phosphatase, acid phosphatase, glucose oxidase, β-galactosidase, β-glucoronidase or β-lactamase. Such enzymes may be used in combination with a chromogen, a fluorogenic compound or a luminogenic compound to generate a detectable signal.

In a preferred embodiment, the detectable agent is a dye, i.e. a molecule detectable by optical means. The dye may be a fluorescent, luminescent or otherwise optically detectable dye.

Preferably, the detectable agent is a fluorescent compound or fluorophore. A "fluorophore", as described herein, can be any small molecule that can re-emit light upon light excitation. Fluorescent compounds or moieties are known in the art and will be readily apparent to one of ordinary skill. Many fluorescent compounds are commercially available with activated groups used to react with other compounds.

The detectable agent is preferably a fluorescent biocompatible dye capable of detection either directly or indirectly in an optical imaging procedure, preferably using visible or near infrared light.

In an embodiment, the fluorescent compound or moiety absorbs and emits light of wavelength 350-850 nm, more preferably 450-800 nm, and most preferably 450-750 nm.

Known fluorescent dyes or fluorophores useful in the present invention include fluorescein, 5-carboxyfluorescein, 6-carboxyfluorescein, fluorescein isothiocyanate (FITC), 6-FAM, rhodamine, rhodamine 6G, rhodamine 123, rhodamine B, Texas Red, tetramethylrhodamine, carboxyrhodamine, carboxyrhodamine 6G, carboxyrhodol, carboxyrhodamine 110, carboxytetramethyrhodamine (TAMRA), tetramethylrhodamine (TMR), tetramethylrhodamine isothiocyanante (TIITC), sulforhodamine 101, Silicon-rhodamine (SiR), carbocyanine, indocarbocyanine, oxacarbocyanine, thuicarbocyanine, merocyanine, indocyanine green (ICG), coumarin, 7-amino-4-methylcoumarin-3-acetic acid, xanthene, phorphyrin, squaraine, phycoerythrin, PerCP (peridinin chlorophyll-a Protein), PerCP-Cy5.5, JOE (6-carboxy-4',5'-dichloro-2',7'-dimethoxyfluorescein), NED, HEX, Cascade Blue, Cascade Yellow, Lucifer Yellow, Marina Blue, Cyanine dyes (Cy2, Cy3, sulfo-Cy3, Cy3.5, Cy5, sulfo-Cy5, Cy5.5, Cy7, sulfo-Cy7, Cy7.5, Cy-Chrome), Alexa Fluor dyes (AF350, AF405, AF430, AF488, AF500, AF514, AF532, AF546, AF555, AF568, AF594, AF610, AF633, AF647, AF660, AF680, AF700, AF750, AF790), DyLight dyes (DyLight350, DyLight405, DyLight488, DyLight547, DyLight550, DyLight594, DyLight633, Dylight647, Dylight650, Dylight680, Dylight755, Dylight800), ATTO dyes (ATTO390, ATTO425, ATTO430LS, ATTO465, ATTO488, ATTO490LS, ATTO495, ATTO514, ATTO520, ATTO532, ATTO Rho6G, ATTO540Q, ATTO550, ATTO565, ATTO Rho3B, ATTO Rho11, ATTO Rho12, ATTO Thio12, ATTO Rho101, ATTO580Q, ATTO590, ATTO594, ATTO Rho13, ATTO610, ATTO612Q, ATTO620, ATTO Rho14, ATTO633, ATTO647, ATTO647N, ATTO655, ATTO Oxa12, ATTO665, ATTO680, ATTO700, ATTO725, ATTO740), Chromeo dyes (Chromeo 488, Chromeo 494, Chromeo 505, Chromeo 546, Chromeo 642), Abberior STAR dyes (STAR 440SXP, STAR 470SXP, STAR 488, STAR GREEN, STAR 512, STAR 520SXP, STAR 580, STAR ORANGE, STAR 600, STAR 635, STAR 635P, STAR RED), Abberior LIVE dyes (LIVE 510, LIVE 515, LIVE 580), Oregon Green dyes (OG 488, OG 500, OG 514), VivoTag dyes (VivoTag-645, VivoTag-680, VivoTag-S680, VivoTag-S750, VivoTag-800), boron-dipyrromethane (BODIPY) dyes (BODIPY 493/503, BODIPY FL-X, BODIPY FL, BODIPY R6G, BODIPY 493/503, BODIPY 530/550, BODIPY TMR-X, BODIPY 558/568, BODIPY 564/570, BODIPY 576/589, BODIPY 581/591, BODIPY TR-X, BODIPY 630/650-X, BODIPY 650/665-X), Dy dyes (Dy350, Dy370XL, Dy375XL, Dy380XL, Dy395XL, Dy405, Dy415, Dy481XL, Dy485XL, Dy490, Dy495, Dy505, Dy510XL, Dy520XL, Dy521XL, Dy530, Dy547P1, Dy550, Dy555, Dy560, Dy590, DY594, Dy610, Dy615, Dy630, Dy631, Dy633, Dy636, Dy647P1, Dy651, , Dy681, Dy682, Dy700, Dy701, Dy725, Dy730, Dy732, Dy751, Dy752, Dy675, Dy676, Dy677, Dy679, Dy776, Dy780, Dy781, Dy782), HiLyte Fluor dyes (HiLyte Fluor 405, HiLyte Fluor 488, HiLyte Fluor 532, HiLyte Fluor 555, HiLyte Fluor 594, HiLyte Fluor 647, HiLyte Fluor 680, HiLyte Fluor 750), IRDye infrared dyes (IRDye 800CW, IRDye 800RS, IRDye 680RD, IRDye 680LT, IRDye 700DX, IRDye 750, IRDye 650, IRDye 700, IRDye 800), ADS dyes (ADS1065A, ADS1075A, ADS775MI, ADS775MP, ADS775PI, ADS775PP, ADS780HO, ADS780WS, ADS785WS, ADS790WS, ADS795WS, ADS798SM, ADS800AT, ADS815EI, ADS830AT, ADS830WS, ADS832WS, ADS845MC, ADS920MC), IRIS dyes (IRIS 2, IRIS 3, IRIS 3.5, IRIS 5, IRIS 5.5, IRIS 7G), ProSense 680, ProSence 750, analogs thereof, conjugates thereof, and combinations thereof.

Fluorescent dyes which are active in the NIR (near-Infra Red region) are known in biomedical applications. Significant progress has been made on the recent development of NIR dyes (including cyanine dyes, squaraine, phthalocyanines, porphyrin derivatives and BODIPY analogues) with much-improved chemical and photostability, high fluorescence intensity and long fluorescent life. Examples of NIR dyes include cyanine dyes (also called as polymethine cyanine dyes) are small organic molecules with two aromatic nitrogen- containing heterocycles linked by a polymethine bridge and include Cy5, Cy5.5, Cy7 and their derivatives. Squaraines (often called Squarylium dyes) consist of an oxocyclobutenolate core with aromatic or heterocyclic components at both ends of the molecules, an example is KSQ-4-H. Phthalocyanines, are two-dimensional 187t-electron aromatic porphyrin derivatives, consisting of four bridged pyrrole subunits linked together through nitrogen atoms. BODIPY (borondipyrromethane) dyes have a general structure of 4,4'-difluoro-4-bora-3a,4a-diaza-s-indacene) and sharp fluorescence with high quantum yield and excellent thermal and photochemical stability.

Other detectable agents that can be attached to the cycloalkyne include PET, SPECT, and MRI imaging agents. The most widely used agents include branched chelating agents such as di-ethylene tri-amine penta-acetic acid (DTPA), 1,4,7, 10-tetra-azacyclododecane-I,4,7, 10-tetraacetic acid (DOTA) and their analogs for complexation with metals such as Gd and Cu. Chelating agents, such as di-amine dithiols, activated mercaptoacetyl-glycyl-glycyl-gylcine (MAG3), and hydrazidonicotinamide (HY IC), are able to chelate metals like 99mTc and 186Re.

Preferably the detectable agent is a fluorescent dye for microscopy or nanoscopy detection, such as confocal microscopy, STED (stimulated emission depletion) microscopy or nanoscopy. Other high-resolution microscopy such as STORM (stochastic optical reconstruction microscopy), PALM (photo-activated localization microscopy), PAINT (points accumulation for imaging in nanoscale topography) and cryo-EM (cryogenic electron microscopy) may be suitable for the detection of the adduct. When cry-EM or electron microscopy is used, it may be necessary the labeling of the cycloalkyne with nanogold or other suitable labels known in the art for these type of microscopies.

In a preferred embodiment, the detectable agent is a fluorescent dye for STED nanoscopy. Suitable fluorescent dyes for STED nanoscopy are known in the art and include, among others, AF488, DyLight 488, STAR488, ATTO488, FITC, STAR440SX, OG488, Chromeo 488, Chromeo 505, AF532, AF555, Cy 3, AF546, DyLight 550, ATTO565, TMR, TRITC, AF594, ATTO647N, ATTO542, ATTO594, SiR, STAR RED, STAR580, STAR635P, AF568, AF633, ATTO633, ATTO655, ATTO590.

Preferably, the detectable agent is a fluorescent dye having at least one sulfonate group; more preferably 1, 2, 3, 4 or 5 sulfonate groups. The inventors have observed that the presence of this group increases the solubility of the labelled cycloalkyne and allows selective labelling of the cellular membrane.

In a particular embodiment, in addition to the labeled cycloalkyne, a fluorescent dye or fluorophore is further added in step ii), preferably a fluorescent dye for microscopy or nanoscopy detection. The addition of an additional dye or fluorophore will allow localizing the analogue among the different structures of the cell, tissue, organs, organoids or organisms. Suitable fluorophores include those defined above, as well as arylene ethynylene oligomers and polymers, DCVJ, NBD, Coumarin, Prodan, Nile Red Styryl or 3HC, a dapoxyl derivative or the ones disclosed by Demchenko AP et al (2009, Biophysical Journal, vol 9, pp 3461-3470). As used herein, the term "dapoxyl derivative" means a lipophilic dye that absorbs peak light energy of wavelengths in the range of 350-400 nm and emits peak light energy in the range of 490-530 nm. Dapoxyl derivative fluorphores useful in the invention include, without limitation 6-propiony1-2-dimethylaminonaphthalene (prodom), 6-dodecanoyl-2-dimethylaminonaphthalene (laurdan), 6-acryloyl-2-dimethylaminonaphthalene (acrylodan), 6-bromoacetyl-2-dimethylaminonaphthalene (badan), and dapoxyl sulfonic acid. In a particular embodiment the fluorophore is laurdan. In another particular embodiment, when the microscopy used to visualize the compound is any type of high-resolution microscopy as the ones mentioned above, the additional dye is a solvatochromic dye such as for example nile-red (NR12A or NR4A) or a related dye. Solvatochromic dyes used in high-resolution microscopy, will allow the skilled person to ascertain the cholesterol rafts stiffness.

In some embodiments, the compound of formula (I) is administered to the cell population or to the organism first. After a sufficient period of time, the cycloalkyne is administered to the cell population or the organism. The amount of time between administration of the first compound and the cycloalkyne can vary. In some embodiments, the time sufficient for the first compound to be incorporated into the plasma membrane can be within 10 minutes up to 2, 3 or 4 hours. This is followed by administration of the cycloalkyne.

In a particular embodiment, the contacting of the cycloalkyne with the cell population or the cells of the organism is performed at least 5 minutes, at least 10 minutes or at least 15 minutes after the administration of the compound of formula (I). That is, step ii) of the method of the invention is carried out at least 5 minutes, at least 10 minutes or at least 15 minutes after the administration of the compound of formula (I) to the cell population or to the organism. In another particular embodiment, the contacting of the cycloalkyne with the cell population or to the cells of the organism is performed at least one hour after the administration of the compound of the invention. In an embodiment, the contacting of the cycloalkyne with the cell population or the cells of the organism is performed between 15 and 240 minutes.

In another embodiment, the compound of the invention and the cycloalkyne are simultaneously administered to the cell population or to the organism. In a particular embodiment, when the compound of formula (I) is administered to an organism, a period of time of at least 30 minutes, preferably at least 1 hour, or more preferably 2 hours, is waited after administration of the cycloalkyne and before the detection step, to allow the unreacted labeled cycloalkyne to be eliminated. In a particular embodiment, a period of time between 30 and 240 minutes, preferably between 60 and 180 minutes, is waited between the administration of the labeled cycloalkyne and the detection step.

In another embodiment, when the method is performed in an organism, the detectable agent bound to the cycloalkyne may be an infrared emitting probe as the ones described above to allow the skilled person to differentiate the labeled analogue or adduct from the auto fluorescence of the tissues of the organism. Alternatively, the detectable agent can be a radionuclide, in which case, the detection can be performed by PET or MRI of other suitable detecting means.

As disclosed herein, "detecting" includes determining the presence, absence, or amount of an analyte in a sample, and can include quantifying the amount of the analyte in a sample or per cell in a sample. The terms "detect" and "identify" are used interchangeably herein. Thus, within the context of the present invention detecting is used to determine the presence of the detectable agent in the cell population or in the cells or the organism. Therefore, since the detectable agent is bound to the cycloalkyne and, by virtue of the click chemistry reaction the cycloalkyne reacts with the cholesterol analogue (compound of formula (I)) and gives rise to a cholesterol derivative or adduct, the detection of the detectable agent in cell membranes will allow the in situ detection of the cholesterol analogue. Within the context of the present invention, the cholesterol analogue of the invention preserves the physicochemical properties of the natural cholesterol molecule and is therefore suitable for direct characterization of cholesterol distribution and dynamics within the cell membranes of the cell population or the cells or the organism. Therefore, it is understood that, the expression "cholesterol detection" or "detecting cholesterol" or "monitorization of cholesterol" is used indistinctly to refer to the detection or monitorization of the cholesterol analogue or derivative.

Detection techniques that can be employed in the present invention include optical methods, electrochemical methods (voltametry and amperometry techniques), thermal conductivity, mass spectrometry, atomic force microscopy, and radio frequency methods, e.g., multipolar resonance spectroscopy. Illustrative examples of optical methods, in addition to microscopy, both confocal and non-confocal, are detection of fluorescence, luminescence, chemiluminescence, absorbance, reflectance, transmittance, and birefringence or refractive index, e.g., surface plasmon resonance, ellipsometry, a resonant mirror method, a grating coupler waveguide method or interferometry.

In a particular embodiment the detection of the detectable reagent is performed by confocal microscopy or super-resolution microscopies (SRM), preferably by STED microscopy or nanoscopy, including STED fluorescence nanoscopy, STED fluorescence correlation spectroscopy (STED-FCS), line interleaved excitation scanning STED-FCS (LIESS-FCS) and raster imaging correlation spectroscopy (RICS).

The method may optionally be carried out on a native cell population. By "native" is meant that the cell has not been modified prior to performing the method provided herein. In particular, the cell may be native in that the cell population is not subjected to a step of lysis or extraction, e.g., lipid extraction, prior to performance of the method provided herein. Thus, a cell may be native in that all or substantially all of the cells in the cell population are intact. Thus, by native is meant that the cell population has not been chemically or physically modified and is thus chemically and physically native. Optionally, the cell population may be chemically native, i.e. it may be chemically unmodified, meaning that it has not been contacted with a chemical agent so as to change its chemistry.

Optionally, the cell population may be physically native, i.e. it may be physically unmodified, meaning that it has not been modified physically. Freezing and/or thawing are examples of physical modifications.

The cell population may optionally be fixed chemically, e.g., to preserve cells from degradation, and to maintain the structure of the cell and of sub-cellular components such as cell organelles, e.g., nucleus, endoplasmic reticulum, and/or mitochondria. The fixative may, for example, be 10% neutral buffered formalin or 4% paraformaldehyde.

Freezing may optionally be performed, e.g., by contacting the cell population with a suitable cooling medium, such as, dry ice, liquid nitrogen, or an agent that has been cooled in dry ice or liquid nitrogen, e.g., isopentane (2-methyl butane). Frozen cell populations may optionally be stored at, e.g., between about -80 and -4 °C, e.g., at -70 or -20 °C.

The cell population may optionally be stained and/or labeled.

In a particular embodiment, the detection of the detectable agent according to step iii) of the method of the invention is performed in living cells.

The term "living cells" used in this specification is to be understood as cells having the compatibility to carry out a wide range of metabolic (or anabolic) reactions that are only possible if said cells keep their structural entity. Examples of such reactions are generation of energy (ATP), recycling of cell constituents, and cell multiplication. A skilled in the art will understand that, when the method of detection of cholesterol of the invention is performed in an organism, the cells of the organism are living cells.

In another particular embodiment, the detection of the detectable agent is performed in thick tissue sections, such as brain tissue sections. The term thick tissue sections make reference to slices of a tissue, an organ or an organoid, with a thickness typically from about 50 µm to 5000 µm, or more typically from about 500 µm to 2000 µm. In a particular embodiment, it is from about 500 µm to 1000 µm. As related before, the detection of cholesterol within the cell population of the thick section may be performed in living cells or optionally, in fixed cells as disclosed herewith. In a particular embodiment, the cell population of the thick section is fixed in paraformaldehyde before the detection of cholesterol.

In another particular embodiment, the invention refers to the detection or monitorization of cholesterol in enriched-cholesterol nanodomains (also named lipid rafts) within the plasma membrane.

By "enriched-cholesterol nanodomains" or "lipid rafts" is meant the formation of specific ordered nanostructures (between 20-200nm) highly enriched in cholesterol and sphingolipids that laterally segregate membrane receptors within the cell membranes in order to carry out their functions. Lipid rafts have been described to be involved in a wide range of cellular functions as well as in almost all human diseases (i.e., cancer, neurodegenerative diseases, diabetes).

In an additional embodiment, the method of the invention may be used for the monitorization of cholesterol within the plasma membrane, for example monitorization of cholesterol dynamics.

### Diagnostic methods

It is generally known that lipid rafts do represent dynamic structural components of cellular membranes integrating signaling events and regulating cell functioning and that their dysregulation can lead to disease.

In this regard, it has been demonstrated the links between lipid rafts and Alzheimer's disease (AD), as shown in Hicks DA et al (Frontiers in Physiology 2012, vol 3, article 189, doi: 10.3389/fphys.2012.00189), where it is shown how the perturbation of lipid raft integrity can affect various signaling pathways leading to cellular death and AD. Also, it is known that cholesterol dramatically enhances the onset of aggregation Aβ42, thus linking the impairment of cholesterol homeostasis with AD (Habchi J et al., 2018, Nature Chemistry, vol 10, pages 673-683)

In addition, the deregulation of cholesterol homeostasis is an important factor in the development of cancer. More particularly, quantity and structural changes in lipid rafts due to alteration in cholesterol is one of the important characteristics of cancer (Ding X et al., 2019, Am J Cancer Res, vol. 9(2), pp 2019-227), which ultimately affects signal transduction. For example, cholesterol accumulation in mitochondria has been reported in several solid tumors from rat or human hepatocellular carcinoma (HC) cells or primary tumor from patients with HC (Montero J et al., 2008, Cancer Research, vol 68 pp 5246-5256).

Likewise, in lysosomal storage disorders (LSD) accumulation of sphingolipids and cholesterol has been reported, leading to alterations in the lysosomal homeostasis. In this regard, in Nieman-Pick disease (NPC) the alteration in the lysosomal function underlie a secondary accumulation of cholesterol in the mitochondria, which results in mitochondrial dysfunction.

In addition, abnormal cholesterol metabolism in the brain is also associated with many neurodegenerative disorders, such as Parkinson's disease, Huntingston's disease (HD) and amyotrophic lateral sclerosis (ALS); and, more particularly, it has been reported a diminution in the cholesterol proportion in membrane lipids rafts in the brain of subjects with Parkinson's disease (Jin U et al., Experimental Neurobiology, 2019, vol 28(5), pp 554-567)

Therefore, since the method of detection of cholesterol of the invention allows the visualization and monitoring of the cholesterol in cell membranes, it will also be suitable for the diagnosis of diseases related with alterations in the cell membrane composition.

In another aspect, the invention relates to a compound of formula (I), for use in a method of diagnosis *in vivo,* hereinafter the first diagnostic method of the invention, of a cell membrane related disease in a subject in need thereof, wherein the compound of formula (I) is used in combination with a cycloalkyne compound labeled with a detectable agent.

The expression "diagnosis" or derivatives of the words, refers to the identification of the presence or characteristic of a pathological condition. It refers both to the process of attempting to determine and/or identify a possible disease in a subject, i.e. the diagnostic procedure, and to the opinion reached by this process, i.e. the diagnostic opinion. As such, it can also be regarded as an attempt at classification of an individual's condition into separate and distinct categories that allow medical decisions about treatment and prognosis to be made. As the person skilled in the art will understand, such a diagnosis may not be correct for 100% of the subjects to diagnose, although preferred it is. The term, however, requires that a statistically significant part of the subjects can be identified as suffering from a disease. The skilled in the art may determine whether the classification is statistically significant using different statistical evaluation tools well known, for example, by determination of confidence intervals, the p-value determination, Student's-test, the Mann-Whitney, etc. Preferred confidence intervals are at least, 50%, at least 60%, at least 70%, at least 80%, at least 90%) or at least 95%. The p-values are preferably, 0.05, 0.025, 0.001 or lower.

The terms "in vivo" and "cell membrane" have already been defined within the context of the first method of the invention for detecting cholesterol.

The term "cell membrane related disease" or "cell membrane related disorder" refers to any pathology wherein there is a dysfunction of the plasmatic membrane or any of the membranes of the intracellular organelles. In this regard, alteration of lipid membrane concentrations is known to play a role in a variety of diseases. Also, mutations affecting the protein constituents of membranes can cause many of these diseases. Proteins in membranes are classified as receptors, transporters, ion channels, enzymes, and structural components. All these classes are often glycosylated, so that mutations affecting this process may change their function. Almost 30 genetic disorders have been attributed to mutations affecting various proteins involved in the transport of amino acids, sugars, lipids, urate, anions, cations, water, and vitamins across the cell membranes. Mutations in genes encoding mitochondrial membrane proteins in-volved in oxidative phosphorylation can cause neurological and other problems. Membrane proteins are also affected by conditions other than mutations. Examples of cell membrane disorders include without limitation myasthenia gravis, multiple sclerosis, pseudohypoparathyroidism, cell disease, cystic fibrosis, Wilson disease, lysosomal storage disorders (LSD) such as Niemann-Pick disease C or A/B types, Alzheimer's disease, Parkinson, depression and cancer. In addition, excess of cholesterol (e.g., in familial hypercholesterolemia), excess of lysophospholipid (e.g., the venom after biting of certain snakes contains phospholipases), or excess of glycosphingolipids (e.g., in a sphingolipidosis) can affect membrane function.

In another aspect, the invention is directed to an *in vitro* or ex *vivo* method, of diagnosis of a cell membrane related disease, hereinafter, second method of diagnosis of the invention, wherein the method comprises the use of a compound of formula (I) in combination with a cycloalkyne compound labeled with a detectable agent.

In a particular embodiment, the disease to be diagnosed according to the first or second method of diagnosis of the invention is selected from cancer, Alzheimer's disease and/or a Lysosomal storage disorder.

The term "cancer" or "tumour" or "tumour disease", as used herein, refers to a broad group of diseases involving unregulated cell growth and which are also referred to as malignant neoplasms. The term is usually applied to a disease characterized by uncontrolled cell division (or by an increase of survival or apoptosis resistance) and by the ability of said cells to invade other neighboring tissues (invasion) and spread to other areas of the body where the cells are not normally located (metastasis) through the lymphatic and blood vessels, circulate through the bloodstream, and then invade normal tissues elsewhere in the body. Depending on whether or not they can spread by invasion and metastasis, tumours are classified as being either benign or malignant: benign tumours are tumours that cannot spread by invasion or metastasis, i.e., they only grow locally; whereas malignant tumours are tumours that are capable of spreading by invasion and metastasis. Biological processes known to be related to cancer include angiogenesis, immune cell infiltration, cell migration and metastasis. Cancers usually share some of the following characteristics: sustaining proliferative signalling, evading growth suppressors, resisting cell death, enabling replicative immortality, inducing angiogenesis, and activating invasion and eventually metastasis. Cancers invade nearby parts of the body and may also spread to more distant parts of the body through the lymphatic system or bloodstream. Cancers are classified by the type of cell that the tumour cells resemble, which is therefore presumed to be the origin of the tumour.

Examples of cancer or tumor include without limitation, breast, heart, lung, small intestine, colon, spleen, kidney, bladder, head, neck, ovarian, prostate, brain, rectum, pancreas, skin, bone, bone marrow, blood, thymus, uterus, testicles, hepatobiliary and liver tumors. In particular, the tumor/cancer can be selected from the group of adenoma, angiosarcoma, astrocytoma, epithelial carcinoma, germinoma, glioblastoma, glioma, hemangioendothelioma, hepatoblastoma, leukaemia, lymphoma, medulloblastoma, melanoma, neuroblastoma, hepatobiliary cancer, osteosarcoma, retinoblastoma, rhabdomyosarcoma, sarcoma, teratoma, acrallentiginous melanoma, actinic keratosis adenocarcinoma, adenoid cystic carcinoma, adenosarcoma, adenosquamous carcinoma, astrocytictumors, bartholin gland carcinoma, basal cell carcinoma, bronchial gland carcinoma, carcinosarcoma, cholangiocarcinoma, cystadenoma, endodermal sinus tumor, endometrial hyperplasia, endometrial stromal sarcoma, endometrioid adenocarcinoma, ependymal sarcoma, Swing's sarcoma, focal nodular hyperplasia, germ cell tumors, glucagonoma, hemangioblastoma, hemangioma, hepatic adenoma, hepatic adenomatosis, hepatocellular carcinoma, insulinoma, intraepithelial neoplasia, interepithelial squamous cell neoplasia, invasive squamous cell carcinoma, large cell carcinoma, leiomyosarcoma, malignant melanoma, malignant mesothelialtumor, medulloepithelioma, mucoepidermoid carcinoma, neuroepithelial adenocarcinoma, nodular melanoma, papillary serous adenocarcinoma, pituitary tumors, plasmacytoma, pseudosarcoma, pulmonary blastoma, renal cell carcinoma, serous carcinoma, small cell carcinoma, soft tissue carcinoma, somatostatin-secreting tumor, squamous carcinoma, squamous cell carcinoma, undifferentiated carcinoma, uveal melanoma, verrucous carcinoma, vipoma, Wilm's tumor. In a particular embodiment, the cancer is hepatocellular carcinoma.

The term "Alzheimer's disease" or "AD", as used herein, refers to a mental deterioration associated with specific degenerative brain disease that is characterized by senile plaques, neuritic tangles and progressive neuronal loss which manifests clinically in progressive memory deficits, confusion, behavioral problems, inability to care for oneself, gradual physical deterioration and, ultimately, death. Patients suffering Alzheimer's disease are identified using the NINCDS-ADRDA (National Institute of Neurological and Communicative Disorders and the Alzheimer's Disease and Related Disorders Association) criteria: Clinical Dementia Rating (CDR) = 1; Mini Mental State Examination (MMSE) between 16 and 24 points and Medial temporal atrophy (determined by Magnetic Resonance Imaging, MRI) >3 points in Scheltens scale.

"Alzheimer's disease" has been classified according to the following stages defined by NINCDS-ADRDA Alzheimer's Criteria for diagnosis in 1984:
- Definite Alzheimer's disease: The patient meets the criteria for probable Alzheimer's disease and has histopathologic evidence of AD via autopsy or biopsy.
- Probable or prodromal Alzheimer's disease: Dementia has been established by clinical and neuropsychological examination. Cognitive impairments also have to be progressive and be present in two or more areas of cognition. The onset of the deficits has been between the ages of 40 and 90 years and finally there must be an absence of other diseases capable of producing a dementia syndrome. The term "prodromal Alzheimer's disease" or "MCI with probable Alzheimer's disease" refers to patients showing MCI and which are considered as showing high risk for conversion to Alzheimer's disease. Criteria for identifying a patient as probable AD are those as defined by the NINCDS-ADRDA criteria (McKhann G. et al, Neurology 1984, 34: 939-44), namely, dementia established by clinical and neuropsychological examination, progressive cognitive impairment present in two or more areas of cognition, onset of the deficits between the ages of 40 and 90 years and absence of other diseases capable of producing a dementia syndrome. The term "mild cognitive impairment" or MCI is related to a transitional stage of cognitive impairment between normal aging and early Alzheimer's disease. Patients are usually identified as having MCI if they fulfill the Mayo Clinic criteria (Clinical Dementia Rating, CDR = 0.5, they show a medial temporal atrophy (determined by Magnetic Resonance Imaging, MRI) which is higher than 3 points in Scheltens scale, they show a pattern of parietal and/or temporal hypo metabolism in Positron Emission Tomography with 18-fluorodeoxyglucose (PET-FDG) (suggestive of AD).
- Possible or non-prodromal Alzheimer's disease: There is a dementia syndrome with an atypical onset, presentation or progression; and without a known etiology; but no co-morbid diseases capable of producing dementia are believed to be in the origin of it.

In a particular embodiment, the first method of diagnosis is used in a subject having possible or non-prodromal Alzheimer disease. In another embodiment, the first method of diagnosis of the invention is used in a subject with MCI.

In another embodiment, the second method of diagnosis the invention is used in a brain tissue sample of a subject having possible or non-prodromal Alzheimer disease, in another embodiment, the second method of diagnosis of the invention is used a brain tissue sample from a subject with MCI.

In a particular embodiment, the MCI is amnestic MCI. The term "amnestic MCI" or "aMCI", as used herein, refers to a type of MCI, the predominant symptom of which is memory loss. This term has been defined in Petersen et. al. Arch Neurol. 1999 Mar;56(3):303-8.

The term "Lysosomal storage disorder" is used herein to refer to diseases characterized by the accumulation of diverse lipid species in lysosomes. Lysosomal storage diseases (LSDs) are a group of about 50 rare inherited metabolic disorders that result from defects in lysosomal function. Lysosomes are sacs of enzymes within cells that digest large molecules and pass the fragments on to other parts of the cell for recycling. This process requires several critical enzymes. If one of these enzymes is defective, because of a mutation, the large molecules accumulate within the cell, eventually killing it. Non limiting examples of LSDs are Sphingolipidoses, Farber disease, Krabbe disease, Galactosialidosis, Gangliosides: gangliosidoses, Alpha-galactosidase, Fabry disease (alpha-galactosidase A), Schindler disease (alpha-galactosidase B), Beta-galactosidase / GM1 gangliosidosis, Sandhoff disease, Tay-Sachs, Juvenile hexosaminidase A deficiency, Chronic hexosaminidase, Gaucher disease (I-III), Sphingomyelinase, Lysosomal acid lipase deficiency, Niemann-Pick disease (A/B), Sulfatidosis, Metachromatic leukodystrophy, Saposin B deficiency, Multiple sulfatase deficiency, Mucopolysaccharidoses, MPS I Hurler syndrome, MPS I S Scheie syndrome, MPS I H-S Hurler-Scheie syndrome, Type II (Hunter syndrome), Type III (Sanfilippo syndrome), MPS III A (Type A), MPS III B (Type B), MPS III C (Type C), MPS III D (Type D), Type IV (Morquio), MPS IVA (Type A), MPS IVB (Type B), Type VI (Maroteaux-Lamy syndrome), Type VII (Sly syndrome), Type IX (hyaluronidase deficiency), Mucolipidosis, Type I (sialidosis), Type II (I-cell disease), Type III (pseudo-Hurler polydystrophy / phosphotransferase deficiency), Type IV (mucolipidin 1 deficiency), Lipidoses, Niemann-Pick disease (C/D), Neuronal ceroid lipofuscinoses, Type 1 Santavuori-Haltia disease / infantile NCL (CLN1 PPT1), Type 2 Jansky-Bielschowsky disease / late infantile NCL (CLN2/LINCL TPP1), Type 3 Batten-Spielmeyer-Vogt disease / juvenile NCL (CLN3), Type 4 Kufs disease / adult NCL (CLN4), Type 5 Finnish Variant / late infantile (CLN5), Type 6 Late infantile variant (CLN6), Type 7 CLN7, Type 8 Northern epilepsy (CLN8), Type 8 Turkish late infantile (CLN8), Type 9 German/Serbian late infantile (unknown), Type 10 Congenital cathepsin D deficiency (CTSD), Wolman disease, Oligosaccharide, Alpha-mannosidosis, Beta-mannosidosis, Aspartylglucosaminuria, Fucosidosis, Lysosomal transport diseases, Cystinosis, Pycnodysostosis, Salla disease / sialic acid storage disease, Infantile free sialic acid storage disease, Glycogen storage diseases, Type II Pompe disease, Type IIb Danon disease , Cholesteryl ester storage disease.

In a particular embodiment the LSD is Niemann-Pick type A/B or C disease.

The term Niemann-Pick disease makes reference to a group of autosomal recessive lysosomal storage disorders characterized by the accumulation of diverse lipid species in lysosomes. Niemann-Pick type A and B are caused by deficits in the activity of acid sphingomyelinase (ASMase), an enzyme that regulates lysosomal sphingomyelin (SM) homeostasis, while Niemann-Pck type C is caused by mutations in NPC1 and NPC2 genes, resulting in functional defects in the lysosomal proteins NPC1 and NPC2, involved in cholesterol efflux from lysosomes.

The term Parkinson's disease refers to a progressive degenerative disease of the central and peripheral nervous systems. Involvement of the dopaminergic substantia nigra, which underlies the primary motor features of the disease, occurs at a time when the disease is well advanced at a neuropathological level. The primary symptoms are the results of decreased stimulation of the motor cortex and other areas of the brain by the basal ganglia, normally caused by the insufficient formation and action of dopamine, which is produced in the dopaminergic neurons of the brain. The motor features of Parkinson's disease are just one component of a much more wide-spread disorder that causes an abundance of non-motor signs and symptoms, including olfactory dysfunction, REM sleep behavioral disorder (RBD), constipation, depression, and cognitive deficits. Importantly, many of these signs and symptoms can precede the motor symptoms by years to a decade or more.

Therefore, within the context of the first and second methods of diagnosis of the invention, the provided methodology will allow the medical practitioner to visualize any variation in the amount, such as increase or decrease, distribution, localization or diffusion of cholesterol within the cell membranes of the cells of the cell population or the organism as well as trafficking of cholesterol and organization or distribution of lipids rafts. For example, an increase or decrease in the amount of cholesterol in the cell membranes may be indicative the presence of the above related diseases.

The expression "is indicative", "diagnosing", "diagnosis" or derivatives are used herein as equivalent to diagnosis, which has been previously defined and refer to the identification of the presence or characteristic of a pathological condition.

The term "increase" is used when the amount of cholesterol detected, that is, the detection of the detectable agent or the adduct of the invention in the cell membranes of the cell population or of the cells of the organism, is higher than its reference value. The amount of cholesterol detected is considered to be higher than its reference value when it is at least 1.5%, at least 2%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, or more higher than its reference value. The term, "decreased" when the amount of cholesterol detected, that is, the detection of the detectable agent or the adduct of the invention in the cell membranes of the cell population or of the cells of the organism is lower than its reference value. The amount of cholesterol detected is considered to be lower than its reference value when it is at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, or more lower than its reference value.

The term "reference value", as used herein, relates to a predetermined criteria used as a reference for evaluating the values or data obtained from the images or the data collected from a subject. The reference value or reference level can be an absolute value, a relative value, a value that has an upper or a lower limit, a range of values, an average value, a median value, a mean value, or a value as compared to a particular control or baseline value. A reference value can be based on an individual sample value, that is a cell population of an organism, such as for example, a value obtained from a sample from the subject being tested, but at an earlier point in time. The reference value can be based on a large number of samples, such as from population of subjects of the chronological age matched group, or based on a pool of samples including or excluding the sample to be tested.

The reference value according to the first and second methods of diagnosis of the invention can be obtained from one or more subjects who do not suffer from any of the diseases that are being diagnosed (i.e., control subjects).

In a particular embodiment, when the disease is Alzheimer's disease, there is an increase in the detection of the adduct of the invention in the cell membrane of the cell population or of the cells of the organism, compared to a reference value.

In another embodiment, when the disease is Parkinson's disease, there is decrease in the detection of the adduct of the invention in the cell membrane of the cell population or of the cells of the organism, compared to a reference value.

In another embodiment, when the disease is cancer, there is an increase in the detection of the adduct of the invention in the cell membrane of the cell population or of the cells of the organism, compared to a reference value. In another embodiment, when the disease is cancer, there is a decrease in the detection of the adduct of the invention in the cell membrane of the cell population or of the cells of the organism, compared to a reference value.

### Adducts of the invention

In a further aspect, the invention is directed to an adduct obtained by reacting a compound of formula (I) and a cycloalkyne compound labeled with a detectable agent.

In another aspect, the invention refers to the use of an adduct obtained by reacting a compound of formula (I) and a cycloalkyne compound labeled with a detectable agent, for detecting or monitoring cholesterol in the cell membrane in a cell population or organism.

Suitable and preferred cycloalkyne compounds labeled with a detectable agent are as defined herein in relation to the methods and uses for detecting cholesterol of the invention.

### Kits of the invention

Another aspect of the invention is directed to a kit comprising a compound of formula (I) and a cycloalkyne compound labeled with a contrast or detectable agent.

In general, the kit comprises the components such as the compounds of the invention, and may further comprise a solvent for dissolving them, a buffer, an osmoregulator and a microbicide, which are individually packaged or are partly combined and packaged together, and are packed in one container. The cycloalkyne of the invention may be unlabeled or labeled. When the cycloalkyne is unlabeled, it may be labeled before use. If desired, the compounds of the invention may be provided as a solid such as a freeze-dried powder; or they may be provided as a solution prepared by dissolving them in a suitable solvent. Various types of containers may be suitably selected and used. The containers may have a shape suitable for labeling of the compounds of the invention, or may be formed of a light-shielding material depending on the properties of the compounds. For example, the containers may have a shape of vials or syringes capable of facilitating administration to patients. The kit may comprise appliances necessary for diagnosis, such as syringe, fusion set, as well as appliances for use in PET or SPECT apparatus. In general, an instruction booklet is attached to the kit.

In another aspect, the invention refers to the use of said kit for detecting or monitoring cholesterol in the cell membrane of a cell population or an organism.

The invention will be described by way of the following examples, which are to be considered as merely illustrative and not limitative of the scope of the invention.

### EXAMPLES

### Material and Methods

### General chemicals

The following lipids were purchased from Avanti lipids (Alabaster, AL): 1-palmitoyl-2-oleoyl-sn-glycero-3-phosphocholine (POPC), 1,2-dioleoyl-sn-glycero-3-phosphocholine (DOPC), 1,2-dioleoyl-sn-glycero-3-phosphoethanolamine (DOTAP), 1,2-dioleoyl-sn-glycero-3-phosphoethanolamine (DOPE), bodipy-chol (TopFluor-chol) and cholesterol (Chol). Lissamine Rhodamine B 1,2-Dihexadecanoyl-sn-Glycero-3-Phosphoethanolamine (Rho-PE) and Cholera Toxin B conjugated to Alexa Fluor 647 were purchased from ThermoFisher Scientific (Waltham, Massachusetts, Ma). Naphthopyrene (NAP) was purchased from Sigma-Aldrich (St. Louis, MO). Alexa Fluor 488-DBCO (AF 488-DBCO), Alexa-Fluor 546-DBCO (AF 546-DBCO), Alexa-Fluor-647-DBCO (AF 647-DBCO) and sulfo-Cyanine3-DBCO (sulfo-cy3-DBCO) were purchased from click chemistry tools (Scottsdale, AZ).

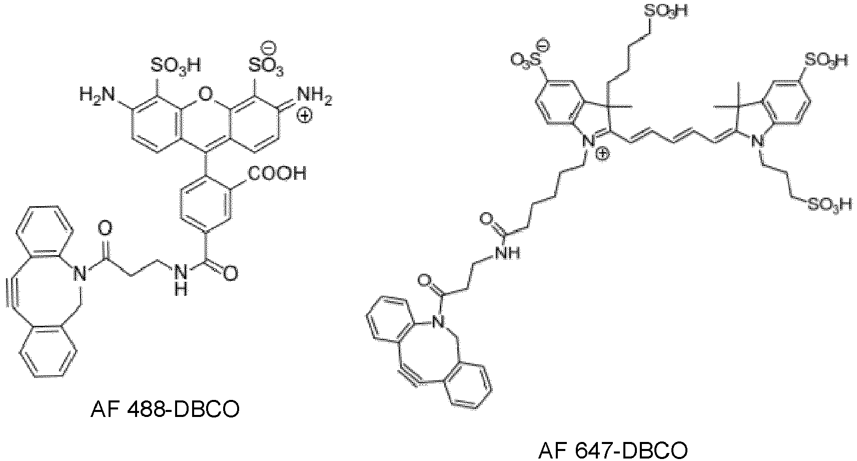

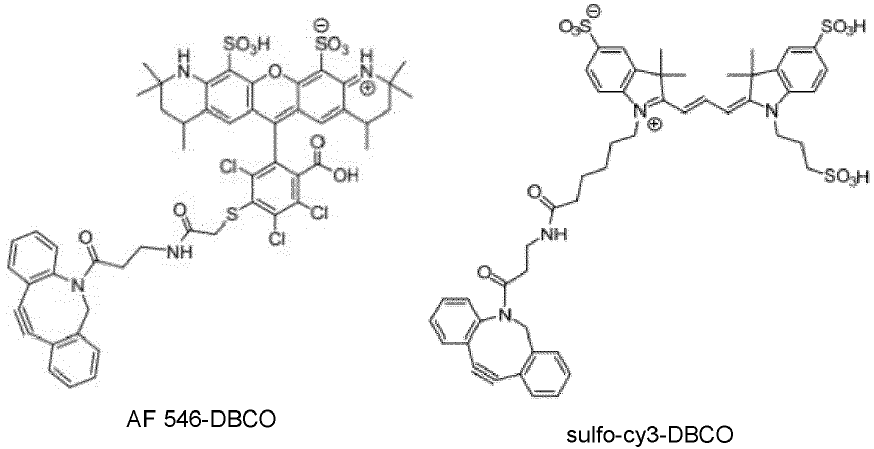

### General procedures

All reagents were obtained from Sigma-Aldrich, Fisher Scientific, or TCI and used without further purification. Anhydrous N,N-dimethylformamide, Tetrahydrofuran, dichloromethane were purchased in sealed bottles and stored over molecular sieves. Deuterated solvents were purchased from Fisher Scientific. Solvents were removed on an IKA Rotavapor RV10 equipped with an ILMVAC LVS210TE vacuum pump. Thin layer chromatography was performed with silica 60 Å gel TLC plates from Merck (60 F₂₅₄) and analyzed by UV illumination or 20% sulfuric acid staining solution. Highpressure NMR spectra were acquired on Bruker AV-500. ¹H NMR spectra were referenced to residual CHCl3 (7.26 ppm). ¹³C NMR spectra were referenced to CDCl₃ (77.16 ppm). NMR spectra were processed using MestReNova (Mestrelab Research S.L.). High-resolution ESI mass spectra of small molecules were obtained at the University of the Basque Country (EHU/UPV) Mass Spectrometry Facility on a Thermo LTQ Orbitrap mass spectrometer.

***Synthesis of 4-(3-((tert-butyldimethylsilyl)oxy)-10,13-dimethyl-2,3,4,7,8,9,10,11,12,13,14,15,16,17-tetradecahydro-1H-cyclopenta[a]phenanthren-17-yl)pentanoic acid (1)***: Compound 1 was synthesized as previously described in Windsor et al. (J Lipid Res 54, 2842-2850 (2013)) with slight modifications. Briefly, in a three-neck bottom flask containing a solution of 3β-hydroxy-5-cholenic acid (0.305 g, 0.814 mmol) in DMF (7 mL) at 0°C was subsequently added Imidazole (0.445 g, 6.54 mmol), DMAP (0.200 g, 1.64 mmol), and TBDMSCI (0.370 g, 2.45 mmol). The solution was stirred overnight at room temperature. After stirring overnight, the reaction was quenched with 1 M HCI (5 mL) and extracted with EtOAc. The organic layer was washed with brine dried over MgSO₄, and the solvent was removed under reduced pressure. Next, the crude product was dissolved in 14 ml MeOH/THF (1/1, v/v) and a 10% (w/w) K₂CO₃ (4 ml) solution was added in water. After stirring for 1h at room temperature, the reaction mixture was concentrated under reduced pressure. The product was resuspended in 25 ml Brine and acidified to pH 3 with 1 M HCI. Finally, the compound was extracted with EtOAc (3x50 ml), dried over MgSO₄ and concentrated in vacuo. The crude product was purified using silica gel chromatography (hexanes: diethyl ether: acetic acid, 70:30:1 v:v:v) to afford a white solid. Yield: 0.345 g (87%). ¹H NMR (500 MHz, CDCl₃) δ = 5.32 (m, 1H), 3.49 (tt, J=10.9, 10.9, 4.7, 4.7, 1H), 2.40 (m, 1H), 2.31- 2.23 (m, 2H), 2.17 (m, 1H), 1.98 (m, 2H), 1.91-1.67 (m, 4H), 1.64-1.24 (m, 10H), 1.20-1.03 (m, 4H), 1.00 (s, 3H), 0.94 (d, J=6.5, 3H), 0.89 (s, 9H), 0.68 (s, 3H), 0.06 (s, 6H). ¹³C NMR (126 MHz, CDCl₃): -4.59 (x2), 11.86, 18.26, 18.29, 19.42, 21.05, 24.25, 25.94 (x3), 28.09, 30.78, 30.90, 31.91 (x2), 32.08, 35.31, 36.58, 37.39, 39.77, 42.39, 42.81, 50.17, 55.75, 56.77, 72.65, 121.12, 141.57, 179.83 ppm. HRMS (ESI) calcd for C₃₀H₅₂NaO₃S: 511.3583 [M+Na]⁺; found: 511.3576.

***Synthesis of 4-(3-((tert-butyldimethylsilyl)oxy)-10,13-dimethyl-2,3,4,7,8,9,10,11,12,13,14,15,16,17-tetradecahydro-1H-cyclopenta[a]phenanthren-17-yl)pentan-1-ol (2):*** Compound 2 was synthesized as described in Kim JH et al., (J Cell Sci 130, 2682-2695 (2017)) with minor modifications. In a 100 ml bottom flask containing a slurry solution of LiAIH4 (0.120 g, 3.16 mmol) in THF (5 ml) at 0°C was added dropwise a solution of (1) (0.340 g, 0.6962 mmol) in THF (20 ml). The reaction mixture was allowed to warm to room temperature and stirred for 1.5h. Next, at 0°C the reaction mixture was quenched with subsequently dropwise addition of H₂O (110 µl), 15% NaOH (110 µl) and H₂O (322 µl). After 1h, the granular salt is filtered off, the organic layer washed with brine, dry over MgSO₄ and concentrated in vacuo. The crude product was purified by column chromatography (hexanes: EtOAc (85/15 v:v) to afford a withe yellowish solid. Yield: 0.265 g (80%). ¹H NMR (500 MHz, CDCl₃) δ = 5.32 (dt, J=4.5, 2.0, 2.0, 1H), 3.49 (m, 1H), 2.40 (m, 1H), 2.27 (m, 2H), 2.17 (m, 1H), 2.02-1.94 (m, 2H), 1.91-1.77 (m, 2H), 1.75-1.64 (m, 1H), 1.63-1.33 (m, 7H), 1.33-1.24 (m, 8H), 1.00 (s, 3H), 0.94 (d, J=6.6, 4H), 0.89 (s, 9H), 0.68 (d, J=1.7, 4H), 0.09 (s, 1H), 0.08 (d, J=0.9, 1H), 0.06 (s, 6H). ¹³C NMR (126 MHz, CDCl₃) δ = 141.72, 121.27, 72.80, 56.91x2, 55.90, 50.32, 42.96, 42.54, 39.92, 37.54, 36.73, 35.46, 32.22, 32.05 (x2), 29.84, 28.24, 26.09 (x3), 24.40, 21.20, 19.57, 18.88, 18.44, 18.41, 12.01, -4.44 (x2). HRMS (ESI) calcd for C₃₀H₅₄O₂SiNa: 497.3791 [M+Na]⁺; found: 497.3783.

***Synthesis of 4-(3-((tert-butyldimethylsilyl)oxy)-10,13-dimethyl-2,3,4,7,8,9,10,11,12,13,14,15,16,17-tetradecahydro-1H-cyclopenta[a]phenanthren-17-yl)pentyl methanesulfonate (3)*.** To a solution of (2) (0.270 g, 0.49 mmol) in dry CH₂Cl₂ (25 ml), neat Et₃N (0.075 g, 0.74 mmol) was added dropwise at 0°C. The resulting mixture was vigorously stirred for 15 min, followed by dropwise addition of neat MsCI (0.085 g, 0.74 mmol). After stirring for 3 h at 0°C, the reaction mixture was diluted by addition of CH₂Cl₂ (10 ml). The organic layer was successively washed with 0.5 N HCI (20 ml), aqueous sat. NaHCO₃ (20 ml), and Brine (20 ml), dried over MgSO₄, filtered and concentrated under reduced pressure. The crude product was purified by flash chromatography (hexane/ethyl acetate 2/1) to afford compound 3 as a white powder. Yield: 0.260 g (96%). ¹H NMR (500 MHz, CDCl₃) δ = 5.32 (m, 1H), 4.23-4.18 (m, 2H), 3.48 (m, 1H), 3.00 (s, 3H), 2.27 (m, 1H), 2.17 (m, 1H), 2.03-1.94 (m, 2H), 1.88-1.76 (m, 3H), 1.74-1.38 (m, 8H), 1.26 (s, 6H), 1.16-1.06 (m, 3H), 1.00 (s, 3H), 0.95 (d, J=6.5, 3H), 0.89 (s, 9H), 0.68 (s, 3H), 0.06 (s, 6H). ¹³C NMR (126 MHz, CDCl₃) δ = 141.57, 121.10, 72.62, 70.66, 56.77, 55.99, 50.16, 42.81, 42.36, 42.34, 39.77, 37.40, 37.38, 36.57, 35.29, 32.08, 31.89, 31.52, 29.69, 28.20, 25.83, 24.23, 22.69, 19.42, 18.55, 11.85, -4.59. HRMS (ESI) calcd for C₃₁H₅₆ NaO₄SSi: 575.3566 [M+Na]⁺; found: 575.3558.

***Synthesis of 17-(5-azidopentan-2-yl)-10,13-dimethyl-2,3,4,7,8,9,10,11,12,13,14,15,16,17-tetradecahydro-1H-cyclopenta[a]phenanthren-3-ol (4)* - *Compound (I), chol-N₃.*** To a three-necked round, bottom flask fitted with a reflux condenser was added a solution of (3) (0.200 g, 0.36 mmol) in anhydrous DMF (5 ml). To the resulting solution, NaN₃ (0.094 g, 1.44 mmol) was added portion wise under argon atmosphere, and the resulting mixture was stirred overnight at 80°C. After cooling down to room temperature, the resulting mixture was quenched by addition of water (20 ml), and the aqueous phase extracted with Et₂O (3x25 ml). The combined organic phases were dried over MgSO₄, filtered and evaporated in vacuo to give a crude mixture, which was purified by gel chromatography (hexanes: diethyl ether: acetic acid, 70:30:1 v:v:v) to afford a white powder. The product was dissolved in 20 ml THF and 4 ml 4 N HCI was added dropwise to the solution. After 2h, the deprotection was stopped by addition of an excess of Na₂CO₃. The reaction mixture was diluted with CH₂Cl₂ (30 ml), washed with 0.1M HCI (20 ml), Brine (20 ml) and dried over MgSO₄, filtered and concentrated under reduced pressure. The crude product was purified by flash chromatography (hexane/ethyl acetate 3/1) to afford compound 4 as a white powder. Yield: 0.120 g (92%). ¹H NMR (500 MHz, CDCl₃) δ = 5.35 (m, 1H), 3.53 (tt, J=11.1, 11.1, 4.6, 4.6, 1H), 3.23 (m, 2H), 2.33 - 2.20 (m, 2H), 1.98 (m, 2H), 1.84 (m, 3H), 1.71 - 1.40 (m, 8H), 1.31 - 0.90 (m, 16H), 0.69 (s, 3H). ¹³C NMR (126 MHz, CDCl₃) δ = 140.74, 121.66, 71.77, 56.71, 55.84, 51.95, 50.07, 42.33, 42.25, 39.73, 37.22, 36.47, 35.44, 32.90, 31.86, 31.61, 28.19, 25.48, 24.23, 21.04, 19.37, 18.62, 18.38, 11.84 ppm. HRMS (ESI) calcd for C₂₄H₃₉N₃NaO: 408.2991 [M+ Na]⁺; found: 408.2995.

### Minimize molecular modeling simulations

Lowest energy structures in vacuum of molecules were computed using CS Chem3D Ultra software employing the MMF94-force field and the steepest-descent algorithm. Minimum RMS gradient was set to 0.1; minimum and maximum move to 0.000001 and 1.0, respectively. Maximum number of interactions moves to 5000.

### In vitro laurdan experiments

Large unilamellar vesicles (LUV) composed of POPC:chol (90%:10%), POPC:chol-N₃ (90%:10%) or POPC/chol-N₃/chol-N₃-AF 647-DBCO (90%:8%:2%) were prepared following the extrusion method described by Cebecauer M. et al. (Membrane Lipid Nanodomains. Chem Rev, 2018). Laurdan was added in the organic phase before solvent evaporation. Laurdan labeled liposomes at a concentration of 30 µM were kept under continuous stirring, and the determination of GP profiles at different temperatures was performed. All fluorescence measurements were made using an SLM Aminco series 2 (Spectronic Instruments, Rochester, NY) spectrofluorimeter as previously described (Sheng R. et al. Nat Commun 3, 1249, 2012). To quantify changes in the Laurdan emission spectrum, generalized polarization (GP) values were calculated as follow: GP = (IB-IR)/(IB+IR), where IB (at 440 nm) and IR (at 490 nm) correspond to the intensities at the blue and red edges of the emission maxima, respectively (Bernardino de la Serna J et al., J Biol Chem 279, 40715-40722, 2004 and Sezgin E et al., Nat Rev Mol Cell Biol 18, 361-374, 2017).

### Electroformation and visualization of Giant Unilamellar Vesicles (GUVs)

Giant unilamellar vesicles (GUVs) were prepared using a homemade chamber that allows direct visualization under the microscope (Goni FM et al., Chem Phys Lipids 218, 34-39, 2019). Briefly, DOPC/SM/chol (3:3:1, mol/mol) or DOPC/SM/chol-N₃ (3:3:1, mol/mol) were prepared to a final concentration of 0.2 mM in chloroform/methanol (2:1, v/v), plus 0.2% Rho-DHPE (marker for L_{d} phases) and 0.5% of NAP (marker for L₀ phases). For *in situ* chol-N₃ labeling, DOPC/SM/chol-N₃ (3/1/1, mol/mol) GUVs containing 0.2% Rho-PE and 1% GM1 were prepared to a final concentration of 0.2 mM. Next, 2 µl of the lipid mixture stock was spread on the platinum wires of the electroformation chamber and organic solvent traces removed by placing the chamber under high vacuum for 45 min. Chamber was then equilibrated at 70°C for 15 min followed by addition of 500 µl of 70°C preheated GUVs formation buffer (10 mM Hepes, pH 7.4). An AC electrical field, 10 Hz, 2.5 Vpp, was then applied for 2 h to the electrodes using a wave generator (TG330 function generator; Thurlby Thandar Instruments, Huntingdon, UK). After switching off the generator, vesicles were left to equilibrate for 30 min at room temperature. GUVs attached to the platinum electrodes were visualized using a Nikon D-eclipse C1 confocal system (Nikon Inc., Melville, NY, USA). For *in situ* analog labeling, 1% of chol-N₃ was labeled *in situ* with AF 488-DBCO for 30 min. After extensive washing, cholera toxin conjugated AF 647 (3 µg/ml) was added to the mixture, which binds GM1 and labels the Lₒ phase.

### Competition experiments

A competition assay was designed to test chol-N₃ and fluorescently pre-labeled chol-N₃ uptake by cells. HeLa cells were seeded onto coverslips in DMEM supplemented with 10% FBS, and co-incubated with a fixed concentration of chol-N₃ (5 µM) or with chol-N₃ (5 µM) labeled with 10 µM AF 488-DBCO and an increasing concentration of natural chol (ranging from 0 to 1 mM) added externally to the cells. After 1h co-incubation of cells with the mixture, cells were stained *in vivo* with 25 µM AF 488-DBCO for 60 min, counterstained with DAPI, fixed, and imaged using a ZEISS apotome. Alternaltively, HeLa cells were treated with 5 µM Bodipy-chol in DMEM supplemented with 10% FBS and co-incubated with increasing concentration of natural chol (0-1mM) for 1h. Cells were fixed, and counterstained with DAPI.

### Cytotoxicity experiments

Cytotoxicity of chol-N₃ was evaluated by measuring LDH release in treated HeLa and primary neuron cells with the CytoTOX 96 Non-Radioactive Cytotoxicity Assay (Promega) according to the manufacturer's instructions. Cells were treated with either 50 µM chol-N₃ (25 µM for neurons) or 0.4% (0.2% for neurons) ethanol in culture medium, or left untreated for 18 h at 37 °C with 5% CO₂. Positive control cell samples were lysed with 10% Lysis Solution for 45' at 37 °C with 5% CO₂. Fresh cell culture medium was used as negative control. Cytotoxicity was calculated as follow: (average sample absorbance at 490nm - average absorbance of the negative control)/(average absorbance of the positive control 490nm - average absorbance of the negative control) x 100.

### Fusogenic liposome preparation

Fusogenic liposomes for effective chol-N₃ delivery onto brain tissue were prepared as previously described in Kleusch, et al. (Molecules 17, 1055-1073 (2012)). Briefly, liposomal lipid components were mixed in chloroform in a molar ratio DOTAP/DOPE/chol-N₃ or DOTAP/DOPE/chol (1/1/0.1). The lipid was deposited as a film on the wall of a glass test tube by solvent evaporation under nitrogen. Solvent traces were removed by placing the sample 1h in a vacuum chamber. Next, the lipid film was suspended in artificial cerebral spinal fluid buffer (ACSF; in mM, NaCl 125, NaHCO₃ 25, KCI 2.5, NaH₂PO₄ 1.25, glucose 25, CaCl₂ 2, MgCl₂) by vortexing at room temperature to form multilamellar vesicles. Finally, homogenization of the sample in an ultrasonic bath for 15 min produces mainly unilamellar vesicles.

### Cell culture and maintenance

Human cervix adenocarcinoma HeLa cells and human bone marrow neuroblast SH-SY5Y cells were obtained from ATCC. HeLa cells were maintained in DMEM (Dulbecco's modified Eagle's medium) supplemented with 10% FBS, 2 mM Glutamax, 100 units/ml penicillin, and 100 µg/ml streptomycin (Life Technologies) at 37 °C with 5% CO₂. SH-SY5Y cells were maintained in DMEM/F12 (F12 and Dulbecco's modified Eagle's medium) supplemented with 10% FBS and 1% penicillin and streptomycin. (Life Technologies) at 37°C with 5% CO₂.

### Ethics Statement

Animal husbandry and all experimental procedures involving rats were approved by the animal ethics committee of the University of the Basque Country and by the local authorities (authorization number: CEEA M20-2018-97 and 99) and were conducted in accordance with the Directives of the European Union on animal ethics and welfare.

### Preparation and maintenance of cultured neurons

Primary cultures of embryonic rat day 18 (E18) (strain Sprague-Dawley) cortical neurons were prepared by using standard procedures; animal dissection was set as DIV0 (Day In Vitro zero). Briefly, cortical cells were dissociated with 0.25% trypsin and 0.1 mg/ml DNAse for 15 min at 37°C, then resuspended by gentle trituration with a Pasteur pipette in MEM (Minimum Essential Medium) with 10% horse serum and 0.6% glucose. 2.5x10⁵ neurons were plated onto poly-L-lysine-coated glass-bottom Petri dishes (Ibidi) or coverslips in MEM-HS-glucose. After 3h, the medium was replaced with Neurobasal A medium containing B27 supplement and 2 mM Glutamax (Invitrogen) and neurons were incubated at 37°C in a humidified incubator with 5% CO₂ during 6 days. At DIV7 and DIV14, 1/5 volume of the medium was replaced with fresh Neurobasal medium with 2% B27. At DIV7 2.5 µM cytosine arabinoside was added to the medium.

### Live-cell imaging

Cortical neurons (DIV14) were seeded onto poly-L-lysine-coated glass-bottom Petri dishes (Ibidi 81158) in media Neurobasal A containing B27 serum. Neurons were labeled with 15 µM chol-N₃ for 16 h at 37°C. Neurons were then washed with 3x1000 µl PBS, then incubated with 25 µM AF 546-DBCO in media for 30 min at 37°C. Finally, cells were extensively washed to remove non-reacted probe and immediately proceed for imaging by confocal microscopy using either a Zeiss LSM880 with Airyscan and a Plan-Aprochromat 63X/1.40 oil objective or a Leica TCS SP5 with a Plan-Apochromat 63x1.30 glycerol objective. Images were further processed using Imaris imaging software (Bitplane).

### STED microscopy

SH-SY5Y cells were seeded onto imaging dishes (Ibidi 81156) in media DMEM/F12 and treated with 15 µM chol-N₃ for 16 h at 37°C. Next day, cells were washed and subjected to *in vivo* labeling with 25 µM AF 488-DBCO for 30 min at 37°C followed by extensive washing (3x1000 µl PBS) to remove unreacted probe and proceed for imaging. Cortical neurons (DIV14) were seeded onto poly-L-lysine-coated imaging dishes (Ibidi 81158) in media (Neurobasal A containing B27 serum). Neurons were labeled with 15 µM chol-N₃ for 16 h at 37°C. Neurons were then washed with 3x 1000 µl PBS, then incubated with 25 µM AF 488-DBCO in media for 30 min at 37°C. Finally, cells were extensively washed to remove non-reacted probe and immediately proceed for microscopy. STED and LSCM images were taken in a commercial LEICA SP8 3X STED SMD confocal microscope (Leica Microsystems, Manheim, Germany). The microscope is equipped with 3 depletion lines and for this work; we used the 592 nm depletion line. The excitation laser beam consisted of a pulsed (80 MHz) super-continuum white light laser (WLL). For a cleaner emission the excitation lines we used a notch filter (NF488) in the optical pathway. The objective employed was a Leica 100x/1.4 NA oil objective. The pinhole was set at one Airy unit. The gated HyD detectors were set with the gated option on and the temporal gated selected was from 2 to 6.5 ns when depleting at 592 nm. Imaging and processing have been done employing either commercial (Leica LAXs, Huygens, SVI) or open source software (ImageJ); analysis and graphing, employing EXCEL (Microsoft) or Origin Pro (OriginLab).

### In vivo Laurdan experiments

Cortical neurons (DIV14) were seeded onto poly-L-lysine-coated glass-bottom Petri dishes (Ibidi 81158) in media Neurobasal A containing B27 serum. Neurons were labeled with 15 µM chol-N₃ for 16 h at 37°C. Neurons were then washed with 3x 1000 µl PBS, then incubated with 25 µM AF 546-DBCO and 2,5 µM Laurdan in media for 30 min at 37°C. Next cells were extensively washed with PBS (3x1000 µl PBS) and imaged on a commercial Leica SP8 microscope.

### LIESS-FCS experiments

SH-SY5Y cells were seeded onto imaging dishes (Ibidi 81156) in media DMEM/F12 and treated with 15 µM chol-N₃ for 16 h at 37°C. Cells were labeled with 25 µM AF 488-DBCO for 30 min at 37°C and extensively washed with PBS. LIESS-FCS scanning was recorded on a commercial Leica SP8 STED microscope using the line step function and alternating the excitation between the confocal and the STED modes between every scanned line. Finally, the obtained intensity data for confocal and STED modes were extracted into two different channels. For this work we used as STED and confocal observation spot size 100 nm and 240 nm, respectively. The values of Dᵣₐₜ =Dₐₚₚ(STED)/Dₐₚₚ (confocal) over space x were obtained as previously described in Schneider, F. et al. (Nano Lett 18, 4233-4240 (2018)).

### RICS experiments

SH-SY5Y cells were seeded onto imaging dishes (Ibidi 81156) in media DMEM/F12 and treated with 15 µM chol-N₃ for 16 h at 37°C. Cells were labeled with 25 µM AF 488-DBCO for 30 min at 37°C and extensively washed with PBS. RICS acquisitions were recorded on a commercial Leica SP8 microscope.

### Acute brain slices experiments

500-1000 µm slices were prepared from adult animals (6 to 7 weeks old) with a Mclllwain tissue chopper and incubated at 30 °C in artificial cerebral spinal fluid (ACSF; in mM, NaCl 125, NaHCO₃ 25, KCl 2.5, NaH₂PO₄ 1.25, glucose 25, CaCl₂ 2, MgCl₂ 1) oxygenated by a 95/5 (v/v) O₂/CO₂ gas mixture. Slices were metabolically labeled, *in situ,* with fusogenic liposome composed of DOPE/DOTAP/chol-N₃ (1/1/0.1) in ACSF for 2 h. After washing, slices were subjected to Copper-free click chemistry labelling using 50 µM cy3-DBCO for 3 h. After extensive washing to remove non-reacted probe, tissue was fixed in 4% paraformaldehyde for 25 min. As a final step, fixed brain tissue was embedded 24 h in a mounting media composed of 70% sorbitol in PBS buffer containing 0.01% sodium azide, which help to align the refractive indexes of the objectives and tissue conferring higher-resolution and -imaging depth. Samples were imaged by Leica Microsystems using a Leica TCS SP5 confocal microscope with the Leica HC Fluotar 10/0.3, HC Apochromat 20/0.77, and HC Apochromat 40/0.75. Image reconstructions were performed using Imaris Imaging software (Bltplane). After imaging, samples were store in PBS containing 0.01% sodium azide at 4°C.

### Statistics

Data were analyzed using SPSS statistics software. Results of Kruskal Wallis test are indicated by: not significant (ns) (p > 0.05), *(p < 0.05), **(p < 0.01), and ***(p < 0.001).

### Results

### 1. Design and characterization of the bioorthogonal based chemical chol smart probe

The chol-N₃ probe (Fig. 1A) bears a small and biologically inert azide group attached to the free end of the chol aliphatic side chain. Upon cell insertion, the azide group reacts *in vivo* with fluorescently-labeled cycloalkynes giving rise to a derivative readily suitable for *in situ* bioimaging of chol.

In order to confirm that the azide group minimally perturbs the biophysical, biochemical, and metabolical properties of chol, minimal energy simulations to compare the stereochemistry and molecular topology of both the probe and its endogenous counterpart were carried out. As Fig. 1B shows, it was found that the absolute stereochemistry of chol-N₃ closely mimics that from chol. Similarly, the three-dimensional structure of the chol probe in a hydrophobic environment exhibited molecular topology and stereochemistry similar to those observed with chol (Fig. 1C). To assess whether the chemical modification introduced in the analog interferes with the native behavior of the natural compound, the biophysical behavior of chol-N₃ to that of chol in model membranes were compared. The fluorescent probe Laurdan was used to detect changes in membrane lateral packing properties due to its ability to sense the polarity of the surrounding environment. To this end, we prepared liposomes of POPC containing Laurdan in the presence of chol, chol-N₃ or chol-N₃-labeled AF 647-DBCO. As observed in Fig. 2, the generalized polarization (GP), which reflects the general membrane order at a given temperature shows the same membrane order behavior irrespective of the chol analog used. Next, the ability of chol-N₃ to induce lipid membrane ordering in model membranes was examined. To this end, Giant Unilamellar Vesicles (GUVs) composed of the ternary mixture POPC/SM/chol (3/3/1 mol%) were prepared. This model membrane system is known to phase separate into liquid-disordered (low chol content) and liquid-ordered (high chol content) domains. The effect of chol-N₃ and natural chol on phase separation behavior was investigated. The fluorescent probes used for Ld and Lo visualization were Rhodamine-PE (0.5%) and naphthalene (0.5%), respectively. As Fig. 3 shows, similar phase separation and domain formation were observed with chol (Fig 3A and 3B) and chol-N₃ (Fig. 3C and 3D). When the labeling experiments were carried out *in situ*, the AF 488-DBCO-labeled chol-N₃ probe showed strong localization in the Lo phase (Fig. 4). Taken together, these results point to similar lateral lipid distribution, chain order, and membrane organization in model membranes containing either chol or its chemical analog.

Next, it was determined whether chol-N₃ and chol follow the same intracellular uptake pathway. To this end, an *in vivo* competition assay in cells where the concentration of chol-N₃ was kept constant while increasing the concentration of free chol in the media was performed. As observed in Fig 5, increasing the levels of extracellular chol gradually blocks the uptake of chol-N₃, which is virtually abolished at 500 µM chol. When the same experiment was repeated with pre-labeled chol-N₃, identical results were obtained to those observed with the unlabeled lipid (Fig 6A), thus confirming that chol-N₃ (labeled or unlabeled) uses the same intracellular uptake pathway than chol *in vivo.* Strikingly, when bodipy-chol was used instead of chol-N₃ (pre-labeled or unlabeled), no competition effect was obtained pointing to a different cellular uptake mechanism between the bodipy-labeled lipid and the parental molecule (Fig 6B). As an additional control, Fig 7 shows that neither chol-N₃ nor the fluorophore-labeled cyclooctyne treatment exert any toxic effect on the cultured cells.

To evaluate the suitability of the chol-N₃ probe for imaging purposes in living cells, chol-N₃-treated cortical neurons were subjected to Cu-free click chemistry with AF 546-DBCO. Confocal imaging of chol-N₃ in living neurons demonstrated an intense chol-N₃ staining which is particullarly robust in the soma, axon, and dendrites (Fig 1D). Strikingly, a strong chol labeling was observed in dendritic spines (Fig 1D, insets i and ii) pointing to a critical role of chol in synaptic plasticity as previously suggested. Labeling of neurons with a series of DBCO-linked fluorescence dyes differing in size and polarity showed a similar labeling pattern, ruling out any possible effect that could be attributed to the fluorescent dye used (Fig 8). Most reassuringly, AF 546-DBCO did not label neurons in the absence of the chol analog (Fig 9). Moreover, treatment of primary neurons with chol-N₃ did not exert any cytotoxic effect (Fig 10).

### Resolving chol nanodomains (lipid rafts) in living cells by STED nanoscopy

Having validated the chol bioorthogonal probe for *in vivo* imaging in living cells by conventional confocal microscopy, it was next tried to combine the chol-N₃ smart probe with SRN to probe nanoscale chol organization within the PM of living cells. To this end, stimulated emission depletion (STED) fluorescence nanoscopy, a broadly used SRN technique for measuring diffusion of lipids in membranes by means of STED Fluorescence Correlation Spectroscopy (STED-FCS) was used. Living SH-SY5Y cells were labeled with AF 488-DBCO and visualized by 2D STED nanoscopy. As Fig 11A shows, chol distribution across the PM of living cells can be observed as punctate nanodomains with a resolution far below the diffraction limit (20-40 nm). Superimposed image of confocal vs. STED nanoscopy clearly shows the improved chol spatial resolution obtained by STED (Fig 11B). Close-up images (Fig 11C-E) proved that the chol probe could be resolved by SRN, thus revealing with great detail the chol-rich nanodomain distribution across the PM of living cells. The increase in chol spatial resolution between STED and confocal microscopy recordings can be calculated from the "Full Width at Half Maximum" (FWHM) in the fluorescent intensity line profiles (panel i) or, alternatively, using a more automatized method based on the lateral width of nanodomains. As shown in Fig 11A, well-defined Gaussian peaks were obtained with both, LSCM and STED, but they were much better resolved when STED nanoscopy was used instead of confocal microscopy (-40 nm versus -200 nm). Quantification of selected FWHM peaks yielded values well below the diffraction limit, reaching a remarkable minimum value of 25 nm. Interestingly, nanodomain size analysis shows that chol-rich nanodomains are heterogeneously distributed through the PM and differ in size, area, and shape (Fig 11G-I). Remarkably, two chol-rich populations: one enriched in small (< 100 nm) nanodomains (57.8 ± 0.5 and 45.6 ± 0.2 for single and double Gaussian Peak, respectively) and one enriched in large nanodomains (>100 nm) (157.8 ± 0.5 for the double Gaussian peak) (Fig 11G) were differentiated. Moreover, domain shape analysis showed that chol-rich nanodomains are heterogeneous with nanodomain populations distributed all along the sphericity value scale (between 0 and 1, been 1 a perfect sphere) (Fig 11 I). When chol-N₃ labeling combined with 2D STED nanoscopy was applied to living primary neurons, chol is also distributed across the neuronal membrane as highly bright punctate nanodomains below the diffraction limit (Fig 12A-C).

Given the extremely high lateral probe resolution obtained using 2D STED, high-definition nanodomain images in the axial plane using chol-N₃ combined with 3D STED nanoscopy in living cells was checked. Chol-rich nanodomains were observed, distributed along the axial plane at many different levels (up to 53 for neuronal membranes) revealing with great detail the distribution of chol across living cell membranes (Fig 11G and 12B). Close-up images in Fig 11J and 12D (inset iv), showed that the chol-rich nanodomain images obtained along the axial plane using 3D STED nanoscopy have much better resolution than those obtained using confocal microscopy. This is the first time that lipid rafts have been directly observed within the membrane of unperturbed living cells with such high-resolution.

### Probing chol-rich nanodomains heterogeneity in living cells

The current view of cellular membranes points to the co-existence of highly-ordered chol-enriched (liquid-ordered) phases and less-ordered chol-depleted (liquid-disordered) phases, where membrane proteins having affinity for one or the other can be laterally segregated across the membrane. To investigate the level of order of the chol-rich nanodomains observed in living cells by SRN, chol-N₃ labeling was combined with a polarity-sensing dye, which partitions into the two phases giving rise to a spectral shift when the dye is localized in a more densely packed lipid membrane. For this purpose Laurdan, a solvatochromic dye that distributes uniformly across the membranes regardless of the lipid lateral packing properties, was used. Thus, the colocalization of chol-N₃ and Laurdan at highly ordered chol-enriched lipid nanodomains would confirm their ordered state. Cortical neurons treated *in vivo* with chol-N₃ and subsequently labeled with AF 488-DBCO combined with Laurdan were imaged (Fig 13A) and the Laurdan generalized polarization (GP) map calculated (Fig 13B). Fig 13B shows that while nonarbitrary differences in GP values across the whole neuron surface were observed, specific neuronal areas displayed higher condensation wherever ordered chol-N₃ nanostructures coexist with Laurdan-stained phases (Fig 13C), depicted as white circles named b and d in insets i, ii, respectively). Strikingly, Fig 13C also shows that chol-enriched nanostructures do not only form nanodomains in ordered areas but also in more disordered regions (depicted as white circles named a and c in insets i and ii, respectively) probing the existence of cholesterol-enriched nanodomains with different degrees of order across membranes. In addition, 3D axon projection and GP quantification of neurons labeled with chol-N₃ and Laurdan showed that the axon membrane, which is enriched in chol, is highly ordered all along the zx planes (1 µM) studied (data not shown).

Then it was checked whether lipid diffusion across chol-enriched membrane nanodomains was slower than free chol diffusion across more fluid domains in living cells. This is a long-standing question in membrane biology that can only be solved by measuring the lipid mobility and, at the same time, determining the ratio between diffusion at large and short length scales (i.e., LSCM vs. STED-FCS). This experimental challenge could be addressed by using chol-N₃ with line interleaved excitation scanning STED-FCS (LIESS-FCS) in living cells as recently described (Schneider F. et al., Nano Lett 18, 4233-4240, 2018). Molecular diffusion dynamics at different spatio-temporal positions could be measured by LIESS-FCS in one single measurement using fast and multiple scanning across a line (alternating STED and confocal laser illumination). The ratio between the diffusion coefficient by STED and confocal yields the Dᵣₐₜ value. As previously reported (Mueller V. et al., Biophys J 101, 1651-1660, 2011, and Schneider F. et al. Mol Biol Cell 28, 1507-1518, 2017), a Dᵣₐₜ value close to 1 indicates free diffusion, whereas, Dᵣₐₜ values <1 are characteristic for trapped molecules and Dᵣₐₜ values >1 suggest active diffusion. Chol diffusion was calculated in live SH-SY5Y cells at different plasma membrane positions (marked as 1, 2 and 3 in Fig 13D) and the corresponding temporal correlation function for each spot was used to build up the correlation carpets in either confocal or STED microscopy (data not shown). Two different diffusion modes were observed for chol across the same zone (framed with a dotted white line). When the chol transit time is similar for small (STED) and large (confocal) spots the parameter Dᵣₐₜ <1, thus suggesting a trapped state (Fig 13). On the other hand, it was also observed a 500 nm region where the chol resident time was smaller in STED mode than in confocal mode (data not shown). In this case, Dᵣₐₜ ≈1, strongly suggesting a free diffusion mode for chol. Overall chol transit time quantification (Fig 13E) showed a chol spatial heterogeneity where chol free diffusion and trapping state modes coexist in the same nanoscale area. These results indicate that the existence of high-ordered and less-ordered lipid nanodomains *in vivo* is highly dependent on the chol content (Fig 13F) and provide valuable insight into the complex heterogeneous nature of cell membranes.

### Spatio-temporal characterization of chol dynamics within the plasma membrane of living cells.

Next, the suitability of chol-N₃ to track macroscopic chol dynamics along the plasma membrane by time-lapse live-cell imaging was checked. For this purpose, living SH-SY5Y cells were treated with chol-N₃ and subsequently the probe was labeled with AF 488-DBCO. Then the chol dynamics across the PM were recorded (data not shown). Remarkably, different dynamic PM processes where chol was directly involved were identified. First, highly dynamic chol nanocluster fusion and fission processes could be continuously observed. Surprisingly, some chol nanoclusters fused rapidly and their mobility was limited for an extended period of time before undergoing fission. Second, during PM tubular biogenesis, chol concentration remained very high and dynamic from beginning to end. Strikingly, highly dynamic chol transfer upon tubular branch fusion events were also recorded. During these chol transfer processes, the concentration of chol increased rapidly in the contact site and the chol pool rapidly diffused across the acceptor tubule towards the PM. These results provide direct visual evidence of chol dynamics and its impact in different processes that account for the extraordinary plasticity of the PM.

To associate this chol dynamics with spatio-temporal changes in chol localization and diffusion rates, chol-N₃ using raster imaging correlation spectroscopy (RICS) was visualized. This technique tracks biomolecule diffusion in living cell membranes on time scales ranging from microseconds to milliseconds. Chol diffusion across the PM was studied in SH-SY5Y cells treated with Chol-N₃ and subsequently labeled with AF 488-DBCO. It was observed that chol distribution is not homogeneous across the plasma membrane. Instead, it is accumulated in nanoclusters displaying high local concentration (data not shown). Different overall slow diffusion coefficients were obtained, depending on whether tubules biogenesis took place or not. Interestingly, when chol diffusion was spatio-temporally resolved at different stages during tubule biogenesis it was observed a high chol recruitment and diffusion in the initial steps of tubule formation followed by a slower chol concentration and diffusion coefficient during and after budding. Once again, these results support the suitability of chol-N₃ to evaluate the role of chol in PM biogenesis, maintenance, and function.

### Long time-lapse nanoscopy of chol dynamics in cells.

Long-time SRN imaging of membrane molecules *in vivo* remains technically challenging due to low-density probe labeling, dye photobleaching, and cellular phototoxicity. Based on the high probe density labeling and photostability of chol-N₃ STED nanoscopy was used to obtain long time-lapse super-resolution imaging of chol dynamics in living cells. Thus, it was possible to record intracellular chol loaded vesicle trafficking and fusion with the PM over the course of 10 min (data not shown). These results open up new avenues for investigation of the role of chol in intracellular membrane trafficking by SRN.

### 3D volumetric mapping of chol distribution in intact tissue

Imaging single lipid species at high resolution in tissues has been difficult, especially in the brain, where large concentrations of lipids and chol can be found. To better understand the impact of chol in brain activity in health and disease, it is not sufficient to fully characterize the organization of chol in single neurons. Instead, a thorough 3D perspective of chol distribution across the entire brain is required. Therefore, it was checked if chol-N₃ could be used above the cellular level or, in other words, if high-resolution images of tissues or even complete organs could be obtained. The starting hypothesis was that given the unique properties of chol-N₃, this probe could help us to visualize the 3D chol organization in thick brain samples by standard fluorescence microscopy without the need to slice the sample into thin serial sections.

For this purpose, thick rat brain slices, varying in thickness from 500 µm to 1 mm, were labeled ex *vivo* with chol-N₃ and subsequently subjected to the Cu-free cycloaddition reaction with sulfo-cy3-DBCO. Fig 14A shows a 3D volumetric reconstruction of thick brain slices where the cells are densely and homogenously labeled throughout the sample. These results are in good agreement with the expected overall distribution of chol-rich brain cells (*i.e.*, oligodendrocytes and astrocytes). To further corroborate the localization of chol-N₃ in the plasma membrane we treated the brain samples with a fluorescently labeled lectin, a well-known plasma membrane marker. A strong colocalization between chol-N₃ and the plasma membrane marker was observed (Fig 14A). On the other hand, in the absence of chol-N₃, only marginal labeling of the tissue was observed (Fig 15), thus confirming the validity of our approach.

Finally, it was determined to what extent the high-resolution visualization of chol-N₃ in deep-tissue structures was possible. To this end, representative sections of rat brain cortex were acquired at high magnification by confocal microscopy. As observed in Fig 14B, 3D volumetric reconstructions of magnified areas were obtained with different objectives and clearly showed the presence of chol-N₃ in brain cells. This is the first report of a bioorthogonal chol analog being used for 3D deep brain chol visualization by confocal microscopy.

In summary, chol-N₃ (1) retains the biophysical and biochemical characteristics of the parental molecule and (2) is compatible with super-resolution nanoscopy. By combining this probe with 2D and 3D STED nanoscopy, it was possible to visualize and characterize chol-rich nanodomains within living cell membranes at a resolution far below the diffraction limit.

Evidences that chol-N₃ is suitable for studies in living cells, including the spatio-temporal characterization of chol dynamics across the PM and the acquisition over extended periods of time-lapse images showing the intracellular chol trafficking by SRN have been provided.

The generation of high-resolution 3D volumetric images showing the distribution of cholesterol through thick brain sections by confocal microscopy highlights the versatility of chol-N₃ for tissue imaging.

## Claims

1. An *in vitro*, *ex vivo* or *in vivo* method for detecting cholesterol in cell membranes comprising:
i) administering to a cell population or to an organism the compound of formula (I) 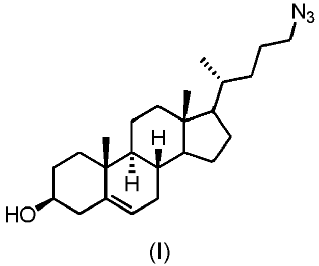 under conditions adequate for the inclusion of said compound in the cell membranes of the cell population or in the cell membranes of the cells of the organism;
ii) contacting the obtained cell population or cells of the organism with a cycloalkyne compound labeled with a detectable agent; and
iii) detecting the presence of the detectable agent in the cell membranes.

2. Method according to claim 1, wherein the cycloalkyne is a cyclooctyne or azacyclooctyne which may include functional moieties such as alkyl, cycloalkyl, aryl, heteroaryl, halogens, ethers, alcohols, ketones, esters, acids, amines, amides, maleimides.

3. Method according to any one of claims 1 or 2, wherein the detectable agent is a fluorescent or luminescent dye, preferably a fluorescent dye for STED microscopy or nanoscopy.

4. Method according to any one of claims 1 to 3, wherein at least steps i) and ii) are performed in living cells.

5. Method according to any one of claims 1 to 3, wherein the detection of cholesterol is performed in tissue sections with a thickness from about 500 µm and 5000 µm.

6. Method according to any one of claims 1 to 5, wherein the cholesterol is in cholesterol-enriched nanodomains within the cell membrane.

7. Method according to any one of claims 1 to 6, for monitoring cholesterol dynamics.

8. Method according to any one of claims 1 to 7, wherein the detection of the detectable agent is performed by confocal microscopy or super-resolution microscopy (SRM), preferably by STED nanoscopy.

9. Method according to any one of claims 1 to 8, wherein the detectable agent is a fluorescent or luminescent dye having at least one, preferably at least two, sulfonate group.

10. Use of a compound of formula (I) 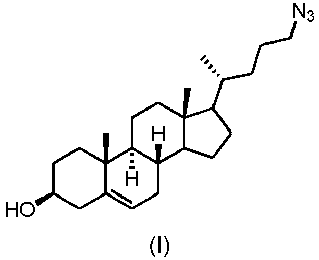 for detecting and/or monitoring cholesterol in cell membranes *in vitro*, *ex vivo* or *in vivo*, wherein the compound of formula (I) is used in combination with a cycloalkyne compound labeled with a detectable agent.

11. A compound of formula (I) as defined in claim 1 for use in a method of diagnosis *in vivo* of a cell membrane related disease in a subject in need thereof, wherein the compound of formula (I) is used in combination with a cycloalkyne compound labeled with a detectable agent.

12. An *in vitro* o *ex vivo* method of diagnosis of a cell membrane related disease, wherein the method comprises the use of a compound of formula (I) as defined in claim 1 in combination with a cycloalkyne compound labeled with a detectable agent.

13. Compound for use according to claim 11 or method according to claim 12, wherein the disease is selected from cancer, Alzheimer's disease, Parkinson's disease and a lysosomal storage disorder; preferably wherein:
- when the disease is Alzheimer, the subject in need of diagnosis suffers from mild cognitive impairment (MCI), or
- the lysosomal storage disorder is Niemann-Pick type A, B or C.

14. An adduct obtained by reacting a compound of formula (I) as defined in claim 1 and a cycloalkyne compound labeled with a detectable agent.

15. A kit comprising a compound of formula (I) as defined in claim 1 and a cycloalkyne compound labeled with a detectable agent.
